# EUROPEAN PATENT SPECIFICATION

(11) **EP 3 908 361 B1**
(45) Date of publication and mention of the grant of the patent: **14.10.2026**
(21) Application number: 19842876.5
(22) Date of filing: 31.12.2019
(51) Int. Cl.: A61N 1/32, A61N 1/40, A61N 5/06, A61N 7/00, A61B 18/12

(54) **DEVICE FOR NON-INVASIVE TREATMENT OF DISEASES AND CONDITIONS OF LIVING ORGANISMS**
VORRICHTUNG ZUR NICHTINVASIVEN BEHANDLUNG VON KRANKHEITEN UND ZUSTÄNDEN LEBENDER ORGANISMEN
DISPOSITIF DE TRAITEMENT NON INVASIF DE MALADIES ET D'AFFECTIONS D'ORGANISMES VIVANTS

(30) Priority: 11.01.2019 HR 20190078
(43) Date of publication of application: 17.11.2021
(73) Proprietor: SYNERGOTRON d.o.o., 10000 Zagreb (HR)
(72) Inventor: PREBEG, Domagoj, 10000 Zagreb (HR); KORDIC, Milan, 10000 Zagreb (HR); LIEBL, Domagoj, 10000 Zagreb (HR); RUDOMINO, Zvonimir, 10360 Sesvete (HR)
(74) Representative: Strniscak, Tomislav
(86) International application number: PCT/HR2019/000031
(87) International publication number: WO 2020/144486

(56) References cited:
- EP-A1- 3 199 201
- US-A1- 2004 034 388
- US-A1- 2016 030 760
- US-B1- 10 039 929

## Description

### FIELD OF THE INVENTION

The present invention is defined by claim 1 and relates to the field of devices for non-invasive treatment of diseases and conditions of living organisms and the methodology of application of such devices for non-invasive treatment of diseases and conditions of living organisms, more specifically, to a device for the synchronous and synergistic use of frequency- and amplitude-modulated pulse profiles, light, electromagnetic field, electric current, dielectric barrier discharge and its constituents, micro-vibrations, sound and with the generation of a therapeutic resonant energy pathway through the target tissue for the purpose of treating diseases and conditions of living organisms.

### TECHNICAL PROBLEM

Modern science confirms that every cell in the human body, of more than 100 trillion cells, generates, absorbs, communicates and responds to various forms of electromagnetic energy and vibration, and that nothing happens in a living organism without electromagnetic or vibrational exchange. There is a constant interaction between our cellular electromagnetism and vibrations, and those imposed from the environment. The principle of resonance implies cellular absorption of the introduced electromagnetic energy and their vibrations or their harmonics. In this way, interfering frequencies can be imprinted into cells, which can be the consequence of toxic substances, traumas, pathogens or prolonged exposure to contamination. Disruption of electromagnetic energy and vibrations can cause damage to cell metabolism and low cellular energy, regardless of the initial cause. The principle of therapeutic application of resonance implies cellular absorption of the introduced electromagnetic energy and vibrations or their harmonics for harmonization, i.e. revitalization of cell metabolism and restoration of cellular energy, physiological function and establishing homeostasis.

Electromagnetism, high-frequency currents and dielectric barrier discharge have been used for more than a century as effective therapeutic factors and energy carriers, which, among many properties, are capable of eliminating pathogens and stimulating innate regenerative processes of living organisms.

The biological effects of electromagnetic fields are attributed to thermal and non-thermal mechanisms. Most thermal effects (heating) include increased metabolic activity and vasodilation with increased perfusion of the treated tissue. The most common non-thermal mechanism for electromagnetic fields and the tissues is the effect of cellular excitation of ion exchange (movement of calcium and other ions) across voltage channels that are sensitive to oscillating electric fields. Disturbances in the function of neuronal calcium signaling are known to be associated with inflammatory and degenerative processes of nerve tissue. Calcium metabolism has also been recognized as an important factor involved in cell regenerative processes. The common use of electromagnetic field stimulation is associated with the treatment of edema, erythema, inflammatory processes, arthralgia, bone and wound healing, pain reduction and an increase in joint mobility.

The therapeutic use of currents on tissues has been shown to activate cellular organelles responsible for cellular activity and cellular energy levels, i.e. to increase the concentration of ATP in cells. Ion channels in the cell membranes can migrate under the influence of electric currents, which leads to cytoskeletal modifications including the formation of membrane projections that allow cell movement. This can facilitate the cell proliferation and protein synthesis, which has been demonstrated in cases where electro-stimulation is being applied to skin, tendons, and cartilage and bone cells. The common use of electrical stimulation is associated with treatment in physical therapy for relaxation, stimulation and rehabilitation of muscles, prevention of atrophy, increase of local blood circulation (arterial, venous and lymphatic flow), maintenance and increase of movement, reduction of chronic, persistent, post-traumatic and postoperative acute pain, immediate post-surgical muscle stimulation to prevent venous thrombosis and promote wound healing.

There are biological molecules that absorb the light of wavelengths in the ultraviolet, visible and infrared portion of the spectrum. These molecules, known as chromophores, act together as little antennas that can receive an electromagnetic wave of a passing photon. If the resonant structure of the molecule corresponds to the photon wavelength, the absorption of the photon is possible. Molecules such as hemoglobin, vitamin B12, cytochrome C and many others are examples of chromophores in human cells. When they absorb the light of a certain wavelength, such molecules activate or produce the energy necessary for their function or activity. These processes are recognized as photoactivation or photobiomodulation. The light absorption in the infrared portion of the spectrum can lead to a local increase in temperature; activations of flavin and cytochrome in the mitochondrial respiratory chain with a consequent effect on electron transport; production of reactive oxygen species through activation of endogenous porphyrins and photoactivation of calcium channels with increasing intracellular calcium concentration and subsequent cell proliferation. The use of ultraviolet light spectrum shows bactericidal properties, improved nucleic acid synthesis and increased oxygen consumption in mitochondria. The common use of photostimulation is associated with the treatment of skin disorders, psoriasis, acne vulgaris, eczema, chronic wounds, and reduction of painful symptoms and stimulation of wound healing.

A dielectric barrier discharge is used in the production of cold atmospheric plasma. Cold atmospheric plasma is an ionized gas that is generated by electromagnetic energy. It consists of resonant electrons, photons and other energized particles that create a unique electromagnetic and micro-vibrational trace. Cold atmospheric plasma has been used for therapeutic purposes for more than a century, and millions of treatments have been performed by such a method because of its antimicrobial and bio-stimulating properties. Studies in the field of plasma medicine indicate obvious interactions between treated tissues and constituents of cold atmospheric plasma. These interactions include antimicrobial effects, control of cellular properties and matrix of tissue, activation of fluids and formation of surface and intracellular electric fields. There is ample evidence that controlled application of cold atmospheric plasma can deactivate dangerous pathogens, including those that are resistant to multiple types of antibiotics, stop bleeding or improve wound healing processes without damaging healthy tissue. The common use of cold plasma is associated with decontamination of skin and wounds from pathogenic microorganisms, promoting wound healing, skin regeneration, and revitalization, treatment of skin diseases and arrest of bleeding.

There is evidence that all living cells generate their micro-vibrations and can absorb externally imposed vibrations, more precisely mechanical or acoustic impulses. Therapeutic use of sound and micro-vibrations is associated with treatment in physical therapy to increase blood supply, lymphatic drainage, and tissue oxygenation, improve cellular metabolism, remove cellular and waste tissue products, reduce swelling and scarring, relieve tissue tension and alleviate painful symptoms.

Inventors believe that resonance, and synchronous and synergistic stimulation with multiple physical factors (entrainment), can be considered as the most important principle explaining the effects of devices and methods of application on living organisms. Resonance can be described as the frequency of vibration that is natural to a particular organ or body. There is a constant interaction between the natural frequencies of the cells and those imposed by the environment. The principle of resonance implies cellular absorption of the applied factors of light, electromagnetic field, electric current, and micro-vibration or their harmonics. Resonance is engaged to coordinate cells that are imprinted with interfering/destructive frequencies that may result from toxic substances, trauma, pathogens or prolonged exposure to contamination.

As each living organism, organ, tissue, cell, molecule or microorganism has a unique electromagnetic imprint, inventors believe that they can be treated with a specific resonant frequency to influence their behaviour. Through the simultaneous and synergistic application of different physical stimuli, more specifically, light, electromagnetic field, electric current, dielectric barrier discharge, micro-vibrations, and sound, with the principle of resonance entrainment can be achieved, i.e. harmonization of disturbed electromagnetism and vibration of cells, in order to increase their energy balance and revitalization at different cellular and tissue levels, or pathogenic microorganisms can be eliminated from the body.

For this purpose, the inventors have developed a device for the application of plasma electro-physical stimulation coupled with resonance for the synchronous and synergistic application of several different physical stimuli, including light, electromagnetic field, electric current, dielectric barrier discharge, micro-vibrations and sound, which can act on different cellular or tissue levels for the purpose of a controlled, expanded and more comprehensive stimulation of the target treated tissue. These factors are generated locally at the site of application, and the electromagnetic field and electric currents allow the non-invasive application of stimulation by establishing a therapeutic resonant energy pathway between the points of application of the device on the surface of the treated organism by acting on deeper located parts of the organism. In doing so, the resonant effects of these factors can be achieved by adjusting the impulse profiles used to generate them. The device also enables real-time monitoring of treated tissue response, dose control, and precise positioning of impulse profile sources during treatment.

### PRIOR ART

Tesla describes in the patent GB189620981A resonance transformer circuits that have made it possible to use naturally oscillating forms of high-frequency electromagnetic energy in medicine. Tesla's circuit is based on coupled primary and secondary transformers, capacitors and a spark. The circuit operates in a cycle with current from the primary transformer connected to the capacitor, which is then discharged through the spark gap. This creates a transient, oscillating current in the primary circuit that causes the high oscillating voltage across the secondary coil. Cold atmospheric plasma can be generated by the high voltage generated across the ends of the secondary coil. The pulse oscillations in Tesla's device last for milliseconds. Each spark across the spark gap produces an impulse of a damped high-voltage sinusoidal waveform over the secondary coil contacts.

Strong in the patent US733343A was first to describe a device containing a vacuum tube having a breakdown electrode in combination with a high-voltage power source.

Floyd describes in the patent US2326773 an applicator that generally contains evacuated tubes with atmospheric air in which electrical discharge can be produced, and the atmosphere inside the tubes is such that it produces ultraviolet radiation (US2326773).

The same principle is described by Mylius in patent WO2006119997A2, WO2006072582A1 in the construction of an ozone-producing therapeutic device that uses partially evacuated tubes with the addition of noble gases.

Korous and Srb describe in the patent WO2014032747A1 a device for treating biological tissue with low-pressure plasma, where the device comprises a transformer for generating a high-frequency electromagnetic field, a probe that can be electrically coupled to a transformer, and a control unit for controlling the high-frequency electromagnetic field generated by the transformer. In accordance with the invention, the probe is connected to an information unit, wherein the unit is used to measure, and optically and/or acoustically displays the duration, output power of the device and/or measures output current power of the device, and if a pre-defined limit value is exceeded, interrupts power supply to the device; and/or measures and displays the optical and/or acoustic distance of the probe treatment surface from the target tissue surface. In the shown setup, it is obvious that no system defines or measures the distance of electrode from the treated tissue surface, and that the system does not include feedback from the treated area, but only the feedback on the current power, i.e. power on the transformer primary.

The prior art also indicates the necessity of electrically driven vibrators and massagers that produce a vibration that stimulates circulation in the affected tissue. Also known in the art are vibration and impact devices that promote bone growth.

For example, in the U.S. patent No. 5,273,028 McLeod describes a device for stimulating bone growth in a living organism by transmitting vertical vibrations through the plate on which the person is located.

In the U.S. patent No. 5,103,806, 5,376,065 and 5,191,880 McLeod also describes a method for preventing osteopenia while promoting bone growth and healing, including broken bones by subjecting the bones to mechanical loading.

In the U.S. patent No. 6,245,006 Olson describes magnetic therapy as an established and reliable technology. In the U.S. patent No. 5,632,720, Kleitz describes a magnetic massage with a motor that, when used, does not come closer than 18 inches to the human body. Therefore, the device does not need to come into physical contact with the body. The wand uses a magnetic field between 950 and 1050 gauss in intensity to facilitate an increase in blood flow.

The U.S. patent No. 6,602,275 reveals to Sullivan the use of scattered photon light waves at 470 nm, 630 nm, and 880 nm to stimulate the process of human healing by reducing the inflammation, stimulating and rebalancing the electromagnetic field surrounding the lives, detox organs, and tissue.

The U.S. patent No. 5,035,235 by Chesky discovers the use of musical sound waves as a therapy for chronic and acute pain.

The U.S. patent No. 5,645,578 by Daffer et al. also describes a therapeutic device that uses musical tones.

In the U.S. patent No. 7335170B2 Milne and Spawr describe a therapeutic micro-vibration massage device, which generates a dynamic magnetic field force and an electromagnetic photon optical light field, followed by acoustic sound penetrating the human body, stimulating an increase in cellular energy, and thereby stimulating a healing effect that reduces or eliminates pain. The device consists of an engine that produces micro-vibrations and sound signals and drives one or more permanent magnets or electromagnets and one or more light sources. The device does not generate high-voltage impulses and high-frequency currents in its operation and is intended solely for local application to the target treated tissue.

Further prior art is known from US 10 039 929 B1, EP 3 199 201 A1, US 2004/034388 A1 and US 2016/030760 A1.

Some of the previous devices use one or two thematic technologies. The main disadvantage of all these therapeutic devices is that they are limited to local/topical stimuli application without the ability to control and adjust impulse profiles during the therapeutic procedure, and which, the inventors believe, plays an important role in the outcome of treatment. More specifically, all of the described devices produce and control only some of the above factors, using fixed impulse profiles, which limits applicability, that is, makes it impossible to apply the resonance principle during treatment. The aforementioned devices also do not have a clearly defined control of energy flow, i.e. real-time dosing and monitoring of treated tissue response during stimulation used during treatment, and which depend on a number of factors including the method of administration and deposition of therapeutic electrodes on the surface of the target treated tissue, conductivity and permeability of the treated tissue as well as other ambient conditions such as humidity and air temperature, which play a significant role in the therapeutic outcome of the treatment and/or condition repeatability of the therapeutic procedure. With this in mind, it is understandable that none of the conventional technologies provides a non-invasive technique for the controlled simultaneous stimulation of a living organism by light, electromagnetic field, electric current, dielectric barrier discharge, micro-vibrations a sound-specific impulse profiles with the possibility of applying the resonance principle for non-invasive treatment of a living organism. Accordingly, in the technical field remains a need for techniques to control energy flow - dosage, modulation of the electromagnetic impulse profile, and monitoring of tissue response to stimulation used during real-time treatment, as well as controlled indirect stimulation by creating a resonant circuit through specific structures, pathways and/or body areas for the purpose of treating various conditions and diseases without introducing a physical invasion of a living organism.

None of the aforementioned devices utilizes or suggests simultaneous, synergistic application of different waveforms of frequency- and amplitude-modulated high-voltage impulse profiles, electromagnetic field, electric current, dielectric discharge, light and micro-vibrations, and do not allow the application of a resonant circuit through the target treated tissue. The prior art is therefore characterized by numerous drawbacks, which are explained in the solutions of the present invention. The solutions of this invention minimize, and, in some cases, eliminate the aforementioned disadvantages, and integrate the technology into a practical hand-held or desktop device. Besides, the solutions disclosed in the present invention offer a more cost-effective production of certain elements, more specifically, transducers, increase the safety and repeatability of the therapeutic procedure and reduce energy consumption both in production and during application of the device itself, compared to the devices shown so far. Conducted lab and clinical studies indicate that the dose, i.e. amounts of energy transmitted, as well as the waveform itself, frequency and amplitude of the output high-voltage signal, current intensity and voltage, transducer position in relation to the target treated surface, as well as the physical properties of the target treated tissue itself, or structures to which the device disclosed by the present invention is applied, play a significant role in the outcome of therapeutic interventions.

### BRIEF SUMMARY OF THE PRESENT INVENTION

The device disclosed by the present invention is configured for applying plasma electro-physical stimulation and the establishment of a therapeutic resonant energy pathway through the target treated tissue for treating diseases and conditions of living organisms. This approach is non-invasive and does not involve physical interference with the treated organism. The therapeutic effect of the device includes increasing the energy balance of the target treated tissue and matching electromagnetism and vibration of cells to their natural frequencies through synchronous synergistic application of physical stimuli to adaptive impulse profiles including light in the infrared, visible and/or UV portion of the spectrum, electromagnetic field, electric current, dielectric barrier discharge, micro-vibrations and sound, and the formation of a therapeutic resonant energy circuit of specific impulse profiles (current, electromagnetic) through specific regions, pathways or tissue structures.

The device for non-invasive treatment of diseases and conditions of living organisms disclosed herein enables the application of frequency- and amplitude-modulated impulse profiles via a pair of therapeutic electrodes that allow simultaneous application of light, EM fields, electric current, micro-vibrations, sound and dielectric barrier discharge by establishing a therapeutic resonant energy pathway through a target treated tissue containing at least one resonant electrode and at least one transducer, wherein the resonant electrode enables the establishment of a therapeutic resonant energy pathway, and the measurement of transmitted energy, voltage, and impedance current during the treatment to control the dose and to adjust the output impulse profile to the generator and impulse profiles controller according to the type and response of the target treated tissue.

The device comprises one or more pairs of therapeutic electrodes, namely transducers and resonant electrodes. One or more transducers are placed directly on the skin or close to the skin of the organism at specific locations, and serve as a source that allows selected stimuli and impulse profiles designed to treat certain diseases or conditions to be applied to a living organism. One or more resonant electrodes are placed directly on the skin or in the immediate vicinity of the skin of the organism at specific locations in such a way that the electromagnetic energy is guided through the treated organism, more precisely by forming a therapeutic resonant energy pathway between one or more transducers and one or more resonant electrodes. In this way, the formation of a therapeutic resonant energy pathway is controlled by the placement of a transducer and a resonant electrode on a living organism, and provides guided stimulation through a specific region (e.g. joint, muscle, limb or abdomen) or via certain conductive pathways in a living organism (e.g. the innervation area of a particular nerve, energy meridians or reflex points). In this way, specific stimuli can be introduced both locally and systematically, i.e. through certain regions of a living organism.

In addition, the resonant electrode serves as a measuring probe that allows real-time monitoring of delivered energy during treatment, i.e. dosing and monitoring of tissue response (voltage, current, impedance) to stimulation used during treatment.

The device acts on the living organism in five basic ways. It should be noted, first of all, that through the target treated tissue located between the transducer and the resonant electrode the electric current (at biological proportions) of a relatively high frequency of high-voltage impulses flows, which allows the formation of a therapeutic resonant energy pathway, whose properties can be adjusted according to the purpose of the therapeutic procedure. In this way, the adaptation of the impulse profiles can result in resonance with different types of tissues for their targeted excitation, focusing, or dispersion of electromagnetic field and applied energy or for the elimination of microorganisms by the principle of mortal oscillating resonance. The second effect creates an electromagnetic field generated by the transducer. The third effect comes from the absorption of light transmitted by the transducer. The fourth effect is created by the dielectric barrier discharge and its constituents, which are created in the space between the active surface of the transducer and the treated surface. All these factors cause micro-vibrations, as a fifth effect, which affect the target treated tissue, while generating dielectric barrier discharge in the field of work, additional sound waves are generated whose properties depend on the characteristics of the high-voltage signal impulse profile used.

The device produces impulse profiles and stimulation factors that are defined by a therapeutic regimen configured to achieve a therapeutic effect for the treatment or suppression of a specific disease or condition of a target treated tissue. Application of the device includes therapeutic treatments: injuries of soft and hard tissue; pain; reflex, trigger and acupressure and acupuncture points and pathways; neurological and neuromuscular disorders and injuries, peripheral neuropathy and circulatory disorder, acute and chronic injuries and disorders of the locomotor system, hyperplasia and neoplasia, inflammations and infections, or elimination of microorganisms.

The therapeutic effects of the present device include revitalization, stimulation of regenerative processes and pain reduction including enhancement of blood and lymph circulation, tissue oxygenation, repolarization of cell membranes, activation of differentiated and undifferentiated cells, reduction of swelling and edema; facilitation of cellular metabolism, energy production, enhanced ion exchange and elimination of metabolites and waste from cells and tissues; reduction of the number of microorganisms, stimulation of the immune response, arrest of bleeding, or pain reduction.

The various embodiments of the devices described in the present disclosure also allow for their minimisation, simplification and cost reduction in manufacturing, control and targeted application of specific light spectra and electromagnetic imprint transmitted to the target treated tissue, and allow control of transducer positioning relative to the surface of the target treated tissue in case of dielectric barrier discharge application, which allows the control of the properties and the increased efficiency of the constituents of the dielectric barrier discharge generated therein, more specifically, enables the adjustment of the distance between the transducer and the surface of the treated organism and the volume at which the discharge is generated. This also reduces the diffusion of cold atmospheric plasma constituents from the site of application to the environment and consequently reduced effective concentration at the site of application, which has a significant effect on the end therapeutic result, especially in the case of microorganism elimination, arrest of bleeding or topical stimulation of the surface layers of target treated tissue by dielectric barrier discharge.

For example, the invention may also be applied to the topical application of physical stimuli of different impulse profiles including light in the infrared, visible and/or UV portion of the spectrum, electromagnetic field, electric current, micro-vibrations, sound and dielectric barrier discharge and its constituents on the skin surface and mucous membranes, or open wounds or exposed body parts, such as subcutaneous tissue, muscle, bone, etc., to eliminate microorganism, stimulate healing and reduce pain.

Furthermore, the invention can be applied to stimulate one or more areas of target tissue of a living organism by placing one or more transducers directly on the skin surface in the projection or in the immediate vicinity of the target treated tissue, and placing one or more resonant electrodes on the skin at the desired point in order to form a therapeutic resonant energy pathway. In that case, the position, i.e. spatial arrangement of the transducers and resonant electrodes in relation to the treated organism, the triggering method (parallel or serial) in the case of applying multiple transducers and resonant electrodes, and the properties of the used impulse profile depend on the topology and purpose of the therapeutic intervention itself. Coupling one or more transducers to one or more resonant electrodes allows the formation of a therapeutic resonant energy circuit through the body between the points of their application. Such an application allows the electromagnetic impulse profiles and stimuli to be directed through specific tissue structures or pathways (nerves, energy meridians, or reflex zones) or areas of the body (joints, muscles, limbs, head, neck, chest or abdomen).

The number of application points of transducers and resonant electrodes can vary from one point to twenty points on a living organism, and the treatments can be applied once or twice a day for an extended period of weeks, months or years. The duration of such a procedure at the point of application can vary from a few seconds (10-60 sec) in the case of applying dielectric barrier discharge up to 30 minutes in the case of applying a resonant circuit through a particular region of a living organism (e.g. knee).

The device for non-invasive treatment of diseases and conditions of living organisms disclosed herein comprises a central unit for the production and control of high-voltage impulse profiles, and one or more pairs of therapeutic electrodes, where each pair of therapeutic electrodes contains one transducer and one resonant electrode, and wherein a central unit is coupled to the referred above one or more pairs of therapeutic electrodes, where the device for non-invasive treatment of diseases and conditions of living organisms generates the course of high-voltage impulse profiles, which are transmitted to the living organism via a pair of therapeutic electrodes, where the transducer acts as a source and the resonant electrode as a sink in the specified system. The central unit may be in the form of a portable hand-held or desktop unit, and it comprises enclosures, a battery or mains power source, a management unit, a control unit, a voltage stabilizer, a block of a generator and a signal profile regulator, which is a block of high-voltage signal profiles. The signal profile generator and controller unit comprises a high-voltage transformer, an energy block connected to the transformer primary, and a measuring block connected to an energy block, a resonant electrode and a management unit, with at least one transducer connected to the secondary of the high-voltage transformer.

The management unit receives a (measurable) voltage signal from the block of the generator and signal profiles regulator, which is generated on the basis of the voltage and current measured within the transducer and the resonant electrode, and on the basis of these measurements, a voltage control signal (modulation) is generated whose amplitude is proportional to the current energy transmitted to the therapeutic resonant energy pathway, and based on monitoring this signal, the control unit can determine in real time whether the disease and condition treatment device is working properly and whether the predicted amount of energy for the particular treatment has been delivered. Waveform voltage parameters on the secondary of the high-voltage transformer of the block of the generator and signal profile regulator are variable and adjustable so that the impulse waveform, amplitude, frequency, amplitude- and frequency modulation in time can be changed and adjusted including the frequency of impulse sequences (impulse frequency), frequency of modulated impulses (resonant frequency) and frequency of modulated signal (amplitude-modulated frequency) and depth of amplitude modulation.

The impulse waveforms that make up the impulse sequence can be square, sinusoidal, trapezoidal, triangular, sawtooth, reversed sawtooth, linear increase (rumpup), linear decrease (rumpdown), exponential growth (rumpup), exponential decay (rumpdown), even harmonics, odd harmonics, exponentially damped sine, exponentially amplified or damped sine, or modulated impulse width.

Besides, the voltage on the secondary of the high-voltage transformer of the block of the generator and the signal profile regulator is modulated by one of the above waveforms, whereby the depth and the frequency of the amplitude modulation are adjustable.

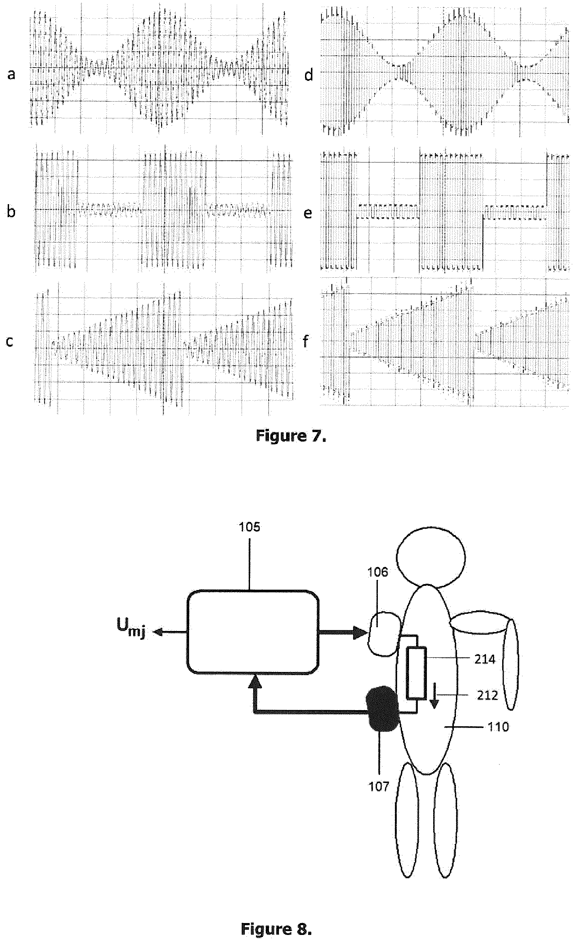

For example, it is possible that the voltage on the secondary of the high-voltage transformer is generated as a sine wave (Figure 7a-c) or as a square (Figure 7d-f) waveform of 43.2 kHz modulated by a sine wave (Figure 7a and d), a square (Figure 7b and e) or a sawtooth (Figure 7c and f) waveform of a control signal voltage (modulation signal) of 2.16 kHz, with a modulation amplitude depth of 80%.

These are just a few examples of the combination of the signal waveform and modulation signal. In doing so, the device can use any combination of the above waveforms for both impulse generation and generation of the voltage control signal (modulation signal).

Different therapeutic impulse profiles consisting of impulse sequences (impulse arrays) that can be regulated and modulated with respect to the waveform (sinusoidal, rectangular, trapezoidal, linear or exponential growth or decay, even and odd harmonics, damped sine), impulse sequence frequency from 0.1 Hz to 7,500 Hz with the frequency modulation in specified range, resonant frequency from 20 kHz to 350 kHz, and voltage amplitude from 500 V to 30,000 V with voltage amplitude modulation by oscillating signal in the range from 0.1 Hz to 5,040 Hz with an adjustable modulation depth can be applied via management unit.

When the transducer is in proximity to a living organism and when the device for non-invasive treatment of diseases and conditions of living organisms is in operation, it enables the formation of a dielectric barrier discharge, generating an electromagnetic field in the frequency range from 40 MHz to 1 GHz and micro-vibration, i.e. a sound in the frequency range from 0.1 Hz to 20 kHz, and a dielectric barrier burst and a mildly ionized cold atmospheric plasma are generated in the air gap between the transducer and the treated tissue surface.When the device for non-invasive treatment of diseases and conditions of living organisms is used to treat the tissue, a transducer- treated tissue- resonant electrode coupling is established, whereby the block of generator and signal profile regulator in real time measures the amount of energy, voltage, current and impedance during treatment implementation, that serves to monitor the amount of energy delivered and the response of the treated organism to stimulation, which depend on the administration modality, the type of target treated tissue and/or ambient conditions during administration allowing real-time control of the amount of energy delivered, by adjusting the output impulse profiles, created by the block of generator and signal profile regulator.

A transducer typically comprises a connector, an insulating housing, a capacitive and/or inductive element, a dielectric barrier, a light source that generates a smoldering discharge in a partially evacuated volume enclosed by dielectric containing one or more gases, which are excited over a dielectric barrier through connectors by means of a capacitive element coupled to the generator and the signal profile regulator. In another embodiment, the transducer comprises a connector, an insulating housing, capacitive and/or inductive element, a dielectric barrier, an electronic circuit with a LED or OLED light source, or an alternative light source in the UV, visible or infrared portion of the spectrum, excited by separate impulse profiles generated by the management unit. The dielectric barrier in the transducer is usually made of glass, ceramics or polymers. The capacitive and/or inductive element is made of conductive material either as a capacitive plate or as a network, or a coil, or a combination thereof, which also may be transparent to light, and is located on the proximal side of the dielectric barrier. The inductive elements contain a contact point for source and sink coupling, the inductive element being directly coupled to the block of generator and impulse profile regulator, and wherein the operation of the device does not require a direct contact with the treated organism, but the transducer and the device are placed at a certain distance from the organism for generating a therapeutic field.

The active surface of the transducer is in the form of a dielectric barrier that is directed or comes into contact with the treated organism, made flat, convex, concave, pointed, or combination thereof, in order to satisfy energy and ergonomic requirements, all depending on the topology and the type and needs of the therapeutic procedure, and is made of glass, ceramic or polymer.

The transducer may further comprise adaptive extensions for precise positioning of the transducer relative to the treated surface of the living organism, it may further comprise a telescopic extension having an inner wall, and may also include a dielectric photo filter, whereby during the operation of the device it is possible to control the discharge properties and to retain constituents generated by the occurrence of dielectric barrier discharge within a closed volume whose walls comprise the active surface of the transducer, the treated surface of the living organism and the inner wall of the telescopic extension, wherein the dielectric photo filter covers the active surface of the transducer transmitting a specific spectrum of light onto the target treated tissue. The adaptive extension may also comprise passive elements adjacent to the active surface of the transducer (506) and the target treated surface, which allow the same distance to be maintained for use on larger surfaces of the living organism.

The resonant electrode comes in contact with the surface of the organism, wherein the said electrode is made of conductive material of arbitrary dimensions and shapes, and is coupled to a central unit, wherein the resonant electrode comprises an electronic circuit that enables voltage measurements and visual or audible signaling to establish a therapeutic energy resonant circuit.

The device for treatment of diseases and conditions disclosed herein generates impulse profiles that include impulse sequences that can be adapted and modulated with respect to their shape, which may be either a sinusoidal, a rectangle, a triangle, a sawtooth or other suitable shape, with impulse frequency ranging from 0.1 Hz to 7,500 Hz, with a resonant frequency ranging from 20 to 350 Hz, with the impulse width ranging from several hundred nanoseconds to 20 microseconds, wherein the amplitude modulation by an oscillating signal is in the frequency range from 0.1 to 5,040 Hz, wherein the impulse amplitude is in the range from around 500 V to 30 kV, and wherein electromagnetic field and high-frequency currents ranging from 40 MHz to 1 GHz and sound waves in the range from 0.1 to 20,000 Hz are generated through the generation of a dielectric barrier in the air gap between the transducer surface and the surface of the target treated tissue. In specific embodiments one or more specific frequencies or sweep over the specified frequency range are used. The depth of the amplitude modulation ranges from 1 to 100%.

Electromagnetic transduction and mechanical transduction are such processes in which cells in a living organism convert electromagnetic and mechanical stimuli into biochemical signals. Tissue and cells can absorb and respond to different types of electromagnetic and mechanical stimuli. The effects of stimulation depend on the type and property of the stimuli. The inventors have found that the simultaneous, harmonised and controlled application of multiple physical stimuli to different pulse profiles including light in the infrared, visible and/or UV portion of the spectrum, electromagnetic field, electric current, dielectric barrier discharge and its constituents, micro-vibrations and sound provides a comprehensive and synergistic stimulation of biochemical signals as well as enhanced effect of cellular entrainment at different cellular and tissue levels, which significantly improves the outcome of the therapeutic intervention. The present invention also enables the application of therapeutic procedures based on the principle of establishing a therapeutic resonant energy circuit through the target treated tissue that allows non-invasive application of various impulse profiles on deep tissue structures placed below, i.e. in the projection of the treated surface of a living organism or through certain conductive pathways below the surface of a living organism including nerves, blood vessels, energy meridians and points.

### BRIEF DESCRIPTION OF THE DRAWINGS

Figure 1a. Two basic derivatives of the device for the application of the plasma electrophysical stimulation and resonance
Figure 1b. A schematic of the therapeutic device
Figure 2. Description of basic control signals
Figure 3. Mode of signals u_{K1}, u_{mod} and Uₙₐₚ
Figure 4. Waveform voltage U_{VN} on a transducer amplitude-modulated by the sinusoidal waveform u_{mod}
Figure 5. U_{VN} voltage on the transducer- increased time axis
Figure 6. Representation of waveform voltage U_{VN} and waveforms of the modulation signal u_{mod}: 1. square, 2. sinusoidal, 3. trapezoidal, 4. triangular, 5. sawtooth, 6. reversed sawtooth, 7.linear increase (rumpup), 8. linear decrease (rumpdown), 9. exponential growth (rumpup), 10. exponential decay (rumpdown), 11. even harmonics, 12. odd harmonics, 13. exponentially damped sine, 14. exponentially amplified sine and 17. modulation of impulse width
Figure 7. Embodiment examples of modulating the sinusoidal and square waveform of a signal by the sinusoidal, square and sawtooth waveforms of the modulation signal u_{mod} (at base signal frequency = 43.2 kHz, modulation signal frequency = 2.16 kHz and amplitude modulation depth (AM) 80%) Figure 8. Measuring the amount of the transferred energy
Figure 9. Electrical equivalent scheme of the system shown in Fig. 8
Figure 10. A view of some typical transducer embodiments
Figure 11. A view of some potential inductive transducer elements
Figure 12. Contact forms of the transducer active surface and the treated surface
Figure 13. Adaptive transducer extension
Figure 14. Administration modalities of the device
Figure 15. A representation of couplings of transducers and resonant electrodes and the establishment of the therapeutic resonant energy pathway through the target treated tissue of a living organism

### A LIST OF REFERENCE SIGNS USED IN DRAWINGS

8- hand-held device
10- device for non-invasive treatment of diseases and conditions of living organisms
112- multichannel source of electromagnetic impulse profiles
100- central unit
101- power supply source
102- management unit
103- control unit
104- power supply stabilizer
105- block of the generator and signal profile regulator
106- transducer
107- resonant electrode
108- therapeutic resonant energy pathway
109- dielectric barrier discharge
110- target treated tissue
204- output impedance R_{gVN}
206- inductance L_{gVN}
208- capacity C_{gVN}
210- transducer impedance Zₜᵣₐₙₛ
212- current that flows through the target tissue impedance Iₜⱼ
214- target tissue impedance Zₜⱼ
216- resonant electrode complex impedance Z_{rez}
218- generator current output I_{VN}
220- generator voltage output U_{VN}
222- signal processing in the measuring block of the generator and signal profile regulator
401- insulating housing
402- capacitive or inductive element
403- smoldering gas discharge
404- dielectric barrier
405- connector for coupling to the generator and impulse profiles regulator (source)
406- retention element
407- electronic circuit with a light source
408- light source (in the UV, visible or infrared portion of the spectrum)
409- connector for coupling of the light source to the management unit
410- connector for coupling of the inductive element sink to the generator and impulse profile regulator
501- adaptive transducer extension
505- retention element on the transducer housing
506- active surface of the transducer
507- telescopic extension
508- dielectric photo filter
509- passive element for maintaining a constant distance when used on larger surfaces
601- single-contact capacitive plate for source coupling
602- single-contact capacitive disc for source coupling
603- single-contact capacitive network for source coupling
604- dual inductive disc with a dual coupling (source-sink)
605- inductive coil with a dual coupling (source-sink)
701, 607- contact point for source coupling (block of the generator and impulse profile regulator)
702, 608- contact point for sink coupling (block of the generator and impulse profile regulator)
700- therapeutic field
801- flat shape of the transducer tip
802- convex shape of the transducer tip
803- concave shape of the transducer tip
804- pointed shape of the transducer tip

### DETAILED DESCRIPTION OF AT LEAST ONE WAY OF CARRYING OUT THE INVENTION

There are two basic embodiments of this device for non-invasive treatment of diseases and conditions of living organisms (10) through the application of plasma electrophysical stimulation and resonance, as a hand-held device (8), which in principle consists of one transducer (106) and one or more resonant electrodes (107), and as a desktop device that consists of a multi-channel source of high-voltage impulse profiles (112) coupled to one or more transducers (106) and resonant electrodes (107). It is a device for synchronous and synergistic use of frequency- and amplitude-modulated impulse profiles, light, electromagnetic field, electric current, micro-vibrations, sound, and dielectric discharge barrier and its constituents with the generation of a therapeutic resonant energy pathway through the target tissue to treat diseases and conditions of living organisms. The present device is non-invasive, and more specifically, does not involve physical penetration of parts of the device into an organism, but is applied solely on its surface or in its immediate vicinity. The device generally consists of a central unit (100) which can be configured as a portable hand-held (8) or desktop device for the production and control of high-voltage impulse profiles, and is coupled to one or more pairs of therapeutic electrodes, more specifically, to a source , i.e. transducer (106) and a sink, i.e. resonant electrode (107). The purpose of the device is to generate a sequence of high-voltage impulse profiles that are transmitted to a living organism via a pair of therapeutic electrodes, with the transducer acting as a source and resonant electrode playing the role of a sink in the aforementioned system.

In one of its embodiments the present device (10) is applied on specific areas of the body using certain impulse profiles that generally include impulse sequences, which can be adjusted and modulated with respect to their shape (sine, right triangle, sawtooth, etc.), frequency of impulse sequences (burst) in the frequency range from 0.1 Hz to 7,500 Hz, through one or more specific frequencies or sweep over the specified frequency range, resonant frequency (of modulated impulses) in the frequency range from 20 to 350 Hz, through one or more specific frequencies or sweep over the specified frequency range, and amplitude modulation (of modulation signal) by an oscillating signal in the frequency range from 0.1 to 5,040 Hz, through one or more specific frequencies or sweep over the specified frequency range, impulse amplitude in the range from about 500 V to 30 kV, and with amplitude modulation depth in the range from 1% to 100%. The treatment also involves the application of an electromagnetic field, high-frequency currents in the range from 40 MHz to 1 GHz and sound waves (0.1 to 20.000 Hz) through the generation of a dielectric barrier discharge in the air gap between the surface of the transducer and the surface of the target treated tissue.

The central unit (100) comprises housings, a battery or mains power source (101), a management unit (102), a control unit (103), a power supply stabilizer (104), and the block of the generator and signal profile regulator (105) , which is in fact a source of high-voltage signal profiles.

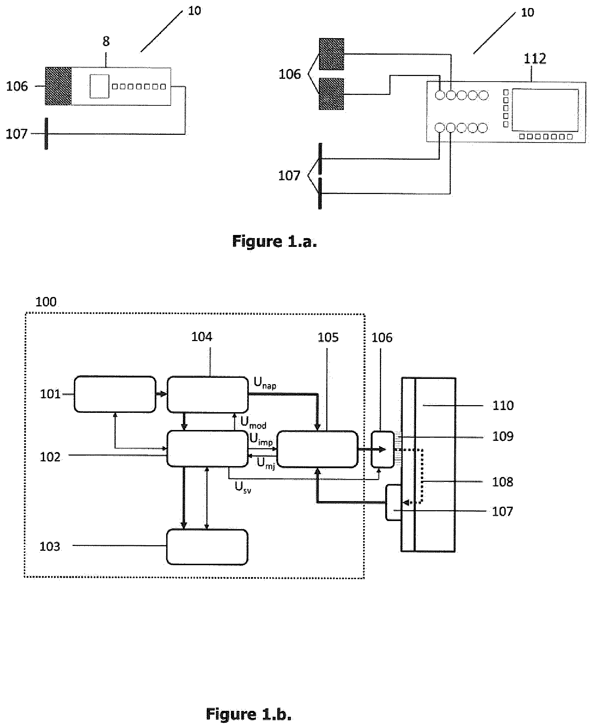

For the proper and efficient operation of the device, it is necessary to ensure the control of the energy amount that is transferred to the treated living organism during treatment. To this end, guidance signals are generated, marked by the arrows in Fig.1 as well as one additional within the controller. In this case, control of the energy flow will mean control over the shape, frequency, amplitude, and duration of the high-voltage impulse profiles, i.e. impulse sequences.

The power supply source, i.e. energy source (101) in Fig.1 may be a galvanically isolated power supply connected to the electrical network or a battery with associated charger and charge control circuits. The power supply stabilizer (104) comprises multiple circuits for generating voltages of different values necessary for supplying other circuits of the system with power. These voltages are fixed in time, but the one indicated in Fig.1 as Uₙₐₚ that serves for supplying the block of the generator and impulse profile regulator (105) with power may also be variable in time. The amount of voltage Uₙₐₚ depends on the managing signal u_{mod} generated by the control unit (102). Variation in the amplitude of this signal will allow variations in power supply voltage Uₙₐₚ, which allows for variations in the amplitude of high-voltage impulses. The amplitude of high-voltage impulses is also affected by the Uᵢₘₚ signal, which controls the energy block at the source of high-voltage impulses. One or more power blocks are connected to one or more high-voltage transformers on whose secondary a transducer is connected. Managing signals are also generated from the management unit block towards the control unit (103), and the management unit (102) also monitors feedback signals from the control unit (103). In this way, it is possible to select stimulation parameters (amplitude, frequency, and amplitude and frequency modulation of signal profiles), to start and stop the stimulation, and to monitor the course of stimulation on the intended display units. The management unit (102) also communicates bidirectionally with the power supply source (101). This achieves battery control, or in the case of mains power supply, control of on-off control systems. The management unit (102) also generates signal U_{SV} that in particular embodiments of the transducer (106) controls the intensity of light therein.

A particularly important signal is the Uₘⱼ received by the management unit (102) from the measuring block (222) of the generator and signal profile regulator (105). It is created based on the voltages and currents measured inside the transducer (106) and the resonant electrode (107). Based on these measurements, the Uₘⱼ signal whose amplitude is proportional to the instantaneous energy that is transmitted to the therapeutic resonant energy pathway (108) is created. Based on the monitoring of this signal, the control unit can determine in real time whether the system is operating as intended and whether the intended dose for the selected treatment has been delivered. Based on this signal, it is possible to change in real time the other aforementioned signals. This achieves the regulation of the energy amount transmitted in a unit of time to the therapeutic resonant energy pathway.

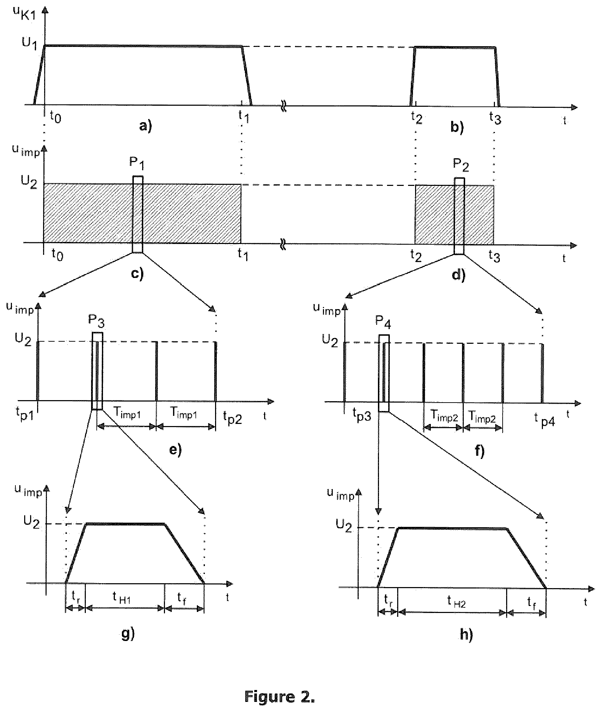

Figure 2 shows basic control signals within the control unit (102). u_{K1} signal at the top of Fig. 2 ( Figures a and b) is the main guiding signal. It is a voltage signal whose activation can be seen as a voltage increase from zero to a value U₁. Activation is initiated by the user himself by starting the system operation on the control unit (103). This is how the sequence of treatment begins that in Fig. 2a lasts from moment t₀ to the moment t₁. The interval between these two moments in absolute amount is determined by the user before activation, by selecting the settings according to the purpose and requirements of the treatment, and is defined by the total amount of the transmitted energy and can vary in the interval from ten seconds to ten minutes. Once the treatment sequence is complete, the device waits for reactivation. It occurs in Fig. 2b at the t₂ moment and the treatment lasts up to the t₃ moment. The treatment duration is not the same as the one from the previous activation, because it was assumed that the user changed the settings of the treatment in the interval between the moments t₁ and t₂. The interval between moments t₁ and t₂ is arbitrary and user-dependent. The user may arbitrarily discontinue the treatment before its expiration on the control unit (103). The layout of the u_{K1} signal will be the same as in Figures 2a and 2b, only the interval between the voltage rise and fall will be smaller.

The high u_{K1} signal level will initiate the generation of the Uᵢₘₚ signal. It is the signal, which manages the block of the generator and signal profile regulator (105) on the transducer shown in Figures 2c and 2d in the time scale from Figures 2a and 2b. To be able to see the characteristics of this signal, it is necessary to increase the time scale. This is done by defining the time windows P₁ and P₂ and their representation in new Figures 2e and 2f with an increased time scale. The arrows connecting the Figures indicate the same points in time.

Uᵢₘₚ signal is actually a sequence of impulses, as seen in Figures 2e and 2f. The impulses are spaced by a time interval Tᵢₘₚ₁, i.e. Tᵢₘₚ, considering that Tᵢₘₚ₁> Tᵢₘₚ so the frequency of occurrence is lower in the former stimulation sequence. The time interval between the impulses is determined by the user via the control unit (103) and is related to the length of treatment. The longer the treatment, the bigger the allowed Tᵢₘₚ value that can be set, i.e., the frequency of the impulse appearance will be smaller, as shown in Figures 2e and 2f. The values of time intervals Tᵢₘₚ, which can be set, range from a few hundreds of nanoseconds to a few tens of milliseconds. During one treatment the Tᵢₘₚ may have a fixed and/or variable value, i.e., it enables frequency modulation.

To be able to more accurately observe the characteristics of uᵢₘₚ signal, it is necessary to increase the time scale. This is done by defining the time windows P₃ and P₄ and their representation in new Figures 2g and 2h with an increased time scale. The arrows connecting the Figures indicate the same points in time. On an enlarged scale, it can be observed that the signal has three separate parts. In the first part, the voltage rises from zero volts to U₂ during rise time tᵣ. It then keeps the voltage value at U₂ in t_{H1} duration and then falls to zero volts at the fall time t_{f}. It is visible in Figures 2g and 2h that the rise times tᵣ and fall times t_{f} are always the same no matter the values of other characteristic times. What the user can change, is the duration of the signal plateau t_{H}.

The duration of the plateau is not related to other times and it is possible to select all the offered t_{H} values in any combination of other parameters. This fact is emphasized in Figures 2e and 2g, as well as in 2f and 2h where it is seen that the shorter Tᵢₘₚ interval does not condition that t_{H} must be shorter. The opposite case has been shown. In one embodiment of the device is the uᵢₘₚ signal, the signal that is transmitted between the gate and the source of the energy MOSFETs in the high-voltage impulse generation circuit. The characteristics of MOSFETs together with the high-voltage transformer and transducer (106) at the output determine the required rise and fall times that are typically in the range of values from a few tens of nanoseconds to a couple of hundred nanoseconds. Since these times are conditioned by the design of the device, there is no reason for them to be under the user's control.

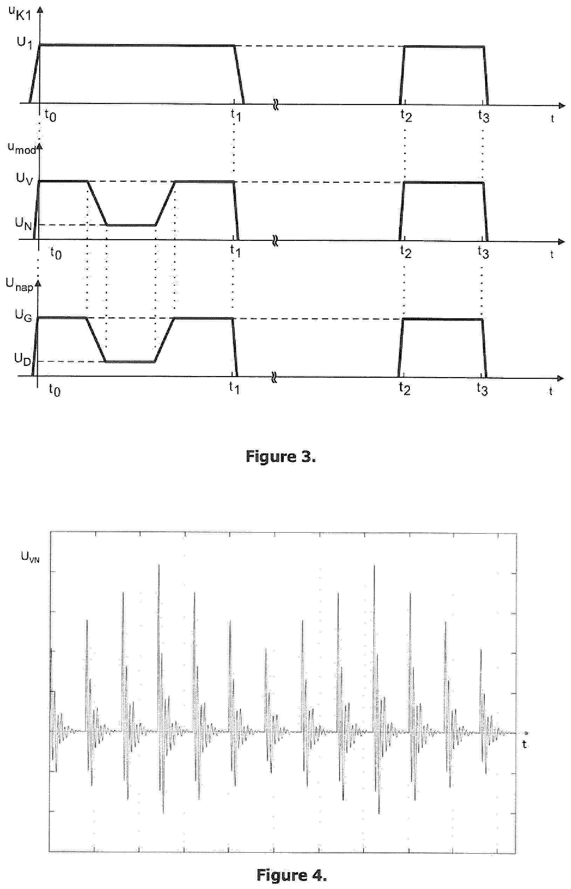

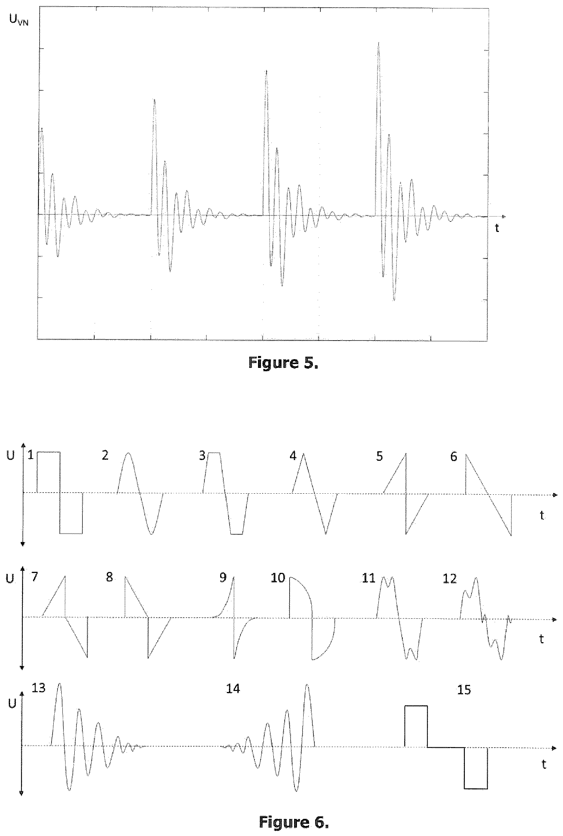

It has already been explained how the signals u_{mod} and Uₙₐₚ are related, and Figure 3 shows in detail, what their temporal relations are to each other as well as to the u_{K1} signal. The Figure shows that the signals u_{mod} and Uₙₐₚ are in time, with the Uₙₐₚ amplitude being directly related to the u_{mod} amplitude with some coefficient, which means that the Uₙₐₚ voltage is amplitude modulated. In Figure 3 the u_{mod} signal has a broken waveform in the interval from t₀ to t₁, which maps to the same one at Uₙₐₚ, meaning that an arbitrary form of voltage can be generated (e.g. rectangle, exponential growth and decay, linear increase and decrease, sine, etc.). Examples of waveforms are shown in Figure 6. The limitation lies in the fact that the voltage changes of u_{mod} are not too fast concerning the final rate of speed change, which may be followed by the voltage stabilizer control loop provided by Uₙₐₚ. For practical implementation, this means that changes can take place for several hundred nanoseconds or more.

Figure 3 also shows that the u_{mod} signal simultaneously serves as an enable signal of the block of the generator and signal profiles regulator (105). The design of the power supply stabilizer (104) enables this, which gives an important safety component to the operation of the device. It should be noted that the Uₙₐₚ signal is an energy signal, which means that the energy portion of the device will remain out of power supply when the u_{K1} signal is inactive. Therefore, even if there is a uᵢₘₚ signal when the u_{K1} signal is inactive due to a fault, the high-voltage impulse source remains without the possibility of energy transfer to the user.

The layout of the energy signal U_{VN} on the transducer (106) in one embodiment of the device, can be seen in Figure 4. This Figure shows the result of the device operating when the u_{K1} signal is active. The Figure shows the use of a sinusoidal waveform of the modulation signal u_{mod}, in the embodiment of a device with a naturally oscillating resonance transformer circuit, more specifically, with an open-core transformer. A more detailed insight into the possible voltage and impulse form in such a device embodiment can be seen in Figure 5. The impulse form can be described by an exponentially damped sinusoidal voltage. The reason for this form lies in the fact that the secondary of the high-voltage transformer together with the secondary parasitic capacity of the transformer freely flickers. The energy is first stored in the transformer, while the MOSFET is switched on in the primary circuit, i.e. by t_{H} (Figures 2g and 2h), and then the transistor shuts off and energy is transferred to the secondary circuit. Since there are losses in the secondary winding in this oscillator, damping occurs in the electrode and in the resistance of the body of the user, which is seen in the exponential decline of oscillation amplitudes with time. It should be emphasized that most of the damping comes from the resistance of the secondary winding, so the voltage waveform will depend a little on the electrical characteristics of the treated living organism. Therefore, the amount of the energy transmitted to the user body will vary slightly (on the order of 10%) on a case-by-case basis. Figure 5 shows an edge case where the distance between individual Tᵢₘₚ impulses (Figures 2e and 2f) is set to start the next impulse at the moment when the energy of the last impulse has already been transferred and consumed.

The moment of MOSFET activation on the primary can easily be observed by the positive voltage spike at the beginning of the waveform shown in Figure 5. The uᵢₘₚ voltage duration is (tᵣ+t_{f}+t_{H}) and can vary from a few hundred nanoseconds up to 20µs. Depending on which transistor is switched on on the primary, the initial spike may be either positive or negative, which also changes the polarity of the voltage of the first period of oscillation, and thus the polarity of the modulated signal envelope (Figure 4), which allows the system operation to be adjusted according to the goals and requirements of the therapeutic intervention.

In another embodiment of the device, the implemented block of the generator and signal profiles regulator (105) has full control over secondary voltage. It also has guidance signals, as described so far, with the difference that the voltage waveform parameters on the transformer secondary (amplitude, frequency, and modulation of frequency and amplitude in time) are variable and adjustable. This means that, at its output, it is possible to obtain both the waveforms of Figures 4 and 5, but also the waveforms shown in Figure 6, except that the waveforms do not depend on the parasitic elements of the secondary circuit of the transformer. The waveforms shown in Figure 6 also represent the waveforms of the u_{mod} modulation signal. Figure 6 shows the waveforms of U_{VN} voltage and the waveforms of the modulation signal u_{mod}: 1. square, 2. sinusoidal, 3. trapezoidal, 4. triangular, 5. sawtooth, 6. reversed sawtooth, 7. linear increase (rumpup), 16. linear decrease (rumpdown), 9. exponential growth (rumpup), 17. exponential decay (rumpdown), 11. even harmonics, 12. odd harmonics, 13. exponentially damped sine, 14. exponentially amplified sine and 15. modulation of the signal width.

Figure 7 shows some of the modulation designs of the sine wave signal Uᵥₙ by sinusoidal (7a), square (7b) and sawtooth (7c) waveforms of the modulation signal u_{mod}, as well as the modulation designs of the square wave Uᵥₙ by sinusoidal (7d), square (7e) and sawtooth (7f) waveforms of the modulation signal u_{mod}. With the ability to select the characteristics of Uᵥₙ and u_{mod}, the described system can adjust the amplitude depth ranging from a just few percents to 100%. In particular, Figure 7 shows an example of the modulation design of a sinusoidal and square waveform of a signal by the sinusoidal, square and sawtooth waveforms of the modulation signal u_{mod} ( at base signal frequency= 43.2 Hz, modulation signal frequency = 2.16 Hz, and amplitude modulation depth (AM) 80%).

The amount of transmitted energy will depend on the duration of the described basic signals. How the energy is transmitted, i.e., how much energy is transmitted in a unit of time will depend on the amplitude and frequency modulation signal and its characteristics. The user does not have complete control over all combinations, but only over those allowed by the controller to avoid possible overuse of energy in the unit of time.

Measuring the amount of the transferred energy is reduced to measuring the target tissue impedance Zₜⱼ (214) and current Iₜⱼ (212) that flows through this impedance as shown in Figure 8.

The block of the generator and signal profile regulator (105) provides energy that passes through the transducer (106), the target treated tissue (110) and the resonant electrode (107).

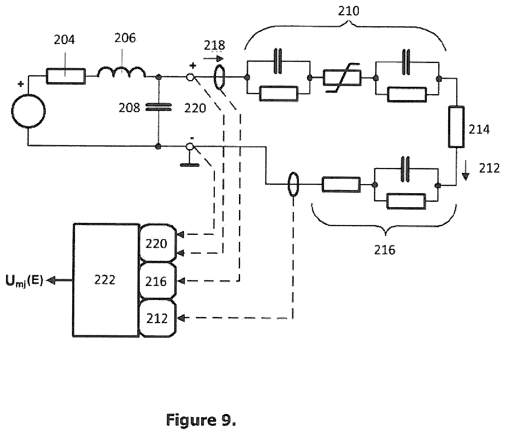

An equivalent electrical scheme of this part of the system is shown in Figure 9. The output circuit of the generator and signal profile regulator is replaced by the ideal voltage source U_{gVN} and by the output passive network of elements. Output impedance R_{gVN} (204) represents the secondary impedance of the high-voltage transformer as well as the mapped impedance of the primary circuit. Inductance L_{gVN} (206) and capacity C_{gVN} (208) in the same way represent the parasitic inductance and secondary capacity of the high-voltage transformer, as well as the mapped inductance and capacity of the primary circuit. It can be said with great certainty that these elements will be linear and independent of the currents and voltages in the network. This does not apply to the transducer impedance Zₜᵣₐₙₛ (210) that has a dominant element of the voltage-dependent nonlinear impedance. The voltage dependence physically arises from the ionization of the low-pressure gas contained within the electrode. The resonant electrode has the same complex impedance Z_{rez} (216), which, however, is predominantly determined by small series impedance. Therefore, it can be assumed that there is practically a short circuit at the stimulation frequency on the electrode. With this assumption, we can say that the current passing through the resonant electrode will be practically equal to the current passing through the target tissue impedance Iₜⱼ (212).

The value of the voltage signal Uₘⱼ is proportional to the transferred energy. To determine this, it is necessary to measure the U_{VN} voltage and the current at the generator output I_{VN} (218) and signal profile regulator, and the reverse current at the output of the resonant electrode Iₜⱼ (212). These two currents differ in value and phase due to the parasitic capacities that exist between all nodes in the equivalent scheme. Based on the measured amplitudes and voltage phases, as well as the amplitude and phase currents, it is possible to calculate the body impedance Zₜⱼ (214). Based on its value, it is also possible to determine the energy transferred to the treated tissue.

From the above considerations, it can be seen that most of the impact on the target tissue will come from the direct effects generated by the current flow and the dielectric barrier discharge along with the transducer. Other effects expected from the electromagnetic field, sound and light will manifest through a minor change in the impedance of the target tissue. Therefore, measuring voltages U_{VN}, I_{VN}, and Itj as accurate as possible is crucial. An extremely high value of interference will be present when measuring due to the immediate vicinity of the generator and signal profile regulator. Several electrostatic shielding measures have been used to minimize the impact of these interferences. When installing the shielding, it was taken into account not to create new parasitic capacitive pathways that would transmit energy from the generator to the mass and that bypass the target tissue. In addition to shielding, a differential signal measurement is used as another way to suppress interference. Interferences also appear here as a single-phase signal at the input of the measurement channel, which is designed for a specially extended single-phase signal travel at the input. Extended signal travel was not sufficient to protect against high amplitude interference, so special attention was given to the circuit protection of the measuring channel. They must meet the opposing requirements for maintaining the large input impedance of the measuring channel and, on the other hand, small physical dimensions that are consistent with the size of electrodes.

The central unit (100) is coupled to one or more transducers (106), i.e. sources that convert electromagnetic impulse profiles into the light, electromagnetic field, electric current, dielectric barrier discharge, micro-vibrations and sound transmitted to the target treated tissue (110). The central unit (100), more specifically, the block of the generator and signal profile regulator (105) is also coupled to one or more resonant electrodes (107), i.e. sinks that enable the establishment of a therapeutic resonant energy pathway (108) through the target treated tissue (110), i.e. between the points of application of the transducer (106) and the resonant electrode (107). The central unit (100), i.e. the block of the generator and signal profile regulator (105) via transducer (106)- treated tissue (110)- the resonant electrode (107) coupling, in real time, measures the amount of energy, more specifically, the voltage, current, and impedance during treatment, thus monitoring the amount of the transmitted energy and the response of the treated organism to stimulation, which depend on the administration modality, the type of target treated tissue and/or ambient conditions during application. This allows for real-time dose control by adjusting the output impulse profiles generated by the block of the generator and impulse profile regulator (105). Impulse profiles and doses for each therapeutic procedure are defined by settings stored in the memory of the management unit (102).

The central unit in the desktop version of the device comprises one or more blocks of the generator and impulse profiles regulator (105) coupled to the management unit (102) on one side and the transducer (106) and the resonant electrode (107) on the other side. In this case, the management unit enables parallel or serial operation ( switching on/off) of individual generators and impulse profile regulators.

The impulse profile settings, more specifically, the impulse sequences and their waveforms and doses are adapted to a particular disease or condition of the treated living organism based on clinical experience, and are stored in the management unit (102), and are defined based on the administration modality, the resonant frequency of the individual tissue or, e.g. the microorganism, and the total amount of the transmitted energy.

The device (10) enables the regular application of various therapeutic impulse profiles, generally consisting of impulse sequences (impulse arrays) that can be regulated and modulated by the management unit (102) with respect to the waveform (sinusoidal, rectangular, trapezoidal, linear or exponential growth or decay, even and odd harmonics, damped sine), impulse sequence frequency from 0.1 Hz to 7,500 Hz, impulse width ranging from several hundred nanoseconds to 20 microseconds with the possibility of frequency modulation in the specified range, a resonant frequency from 20 kHz to 350 kHz, and a voltage amplitude of 500 V to 30,000 V with voltage amplitude modulation by oscillating signal in the range from 0.1 Hz to 540 Hz with adjustable modulation depth.

The device (10) also enables the application of a dielectric barrier discharge when the transducer is in immediate vicinity to a living organism generating an electromagnetic field in the frequency range from 40 MHz to 1 GHz and micro-vibrations, i.e. sound in the frequency range from 0.1 Hz to 20 kHz, also a dielectric barrier discharge together with a slightly ionized cold atmospheric plasma are generated in the air gap between the transducer and the surface of the treated tissue.

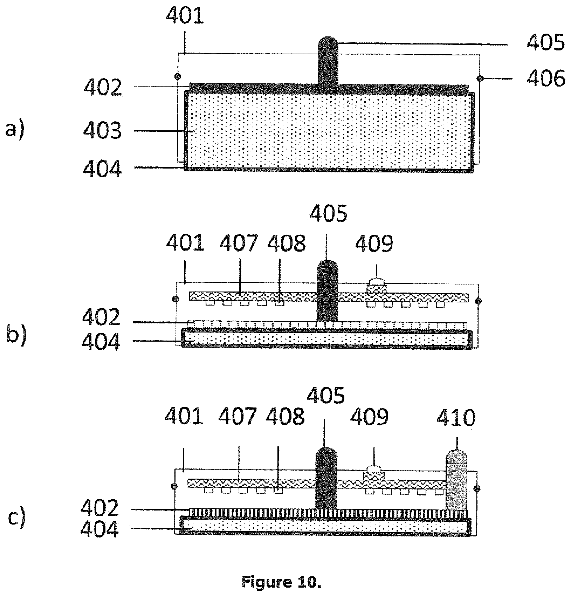

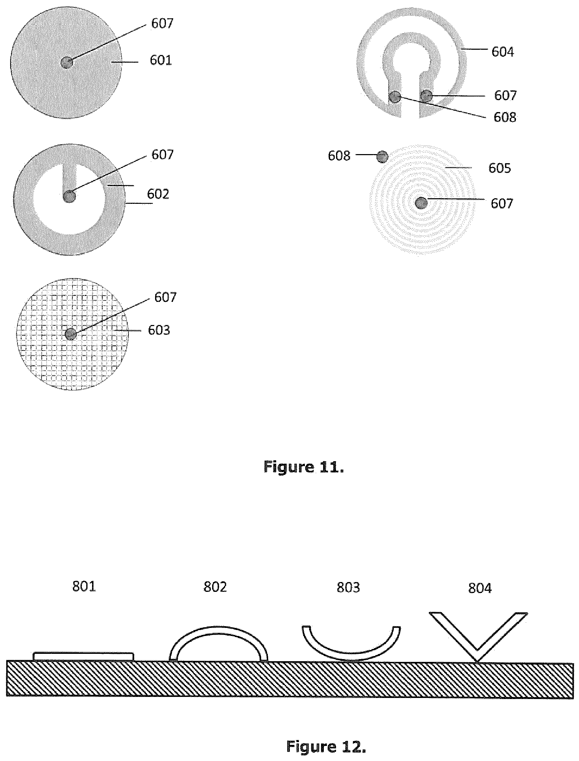

The transducer (106), as shown in Figure 10, in principle, contains an insulating housing (401), a capacitive and/or inductive element (402), a dielectric barrier (404) made of glass, ceramics or polymers, and a light source generated by a smoldering discharge in a partially evacuated volume enclosed by dielectric containing one or more gases (403), which by means of the capacitive element (402) (601 in Figure 11) coupled to the block of the generator and signal profile regulator (105) are excited over a dielectric barrier (404) through the connector (405), or the light generates a separate electronic circuit (407) with a light source (408) such as LED, OLED or any other alternative light source in the UV, visible, or infrared portion of the spectrum, excited by separate impulse profiles generated by the management unit (102). Also, Figure 10 shows a connector for the coupling of the light source to the management unit (409) and the connector for the coupling of the inductive coil sink to the generator (410).

Capacitive and/or inductive element (402), shown in Figure 10, can be made of conductive material as a capacitive plate (601), a capacitive disc (602) or as a capacitive network (603), an inductive disc (604) or a coil (605) or combination thereof, which may be transparent to light, as shown in Figure 11, and is located on the proximal side of the dielectric (opposite side of the active surface of the transducer).

A coil with a dual coupling (605), shown in Figure 11, is made of conductive material and comprises a contact point for source coupling (607) and a contact point for sink coupling (608). A coil (605) can be flat or conically wound and arrangement and length of coil are of arbitrary width/thickness of wire.

In the embodiments of the transducer containing inductive elements (604), (605), as shown in Figure 11, this element contains a contact point for source coupling (607) and sink coupling (608), and is directly coupled to the block of the generator and signal profile regulator (105), and their operation is possible without a direct contact with the treated organism, whereas the transducer (106) and the device (10) are positioned at a certain distance from the organism to generate a therapeutic field. Some of the possible embodiments of the transducer with inductive elements are a dual inductive disc with a dual coupling (source-sink) (604) or a coil with a dual coupling (source-sink) (605). In the case where the transducer contains capacitive elements, some possible embodiments are a single-contact capacitive plate for source coupling (601), a single-contact capacitive disc for source coupling (602) and a single-contact capacitive network for source coupling (603).

The active surface of the transducer (106), i.e. the dielectric barrier (404), which is directed or comes into contact with the treated organism, can vary in shape and size, all as shown in Figure 12, wherein the shape is flat (801), convex (802), concave (803) or pointed (804), or combinations thereof, all to meet energy and ergonomic requirements depending on the type and needs of the therapeutic procedure, and is also made of glass, ceramics or polymers.

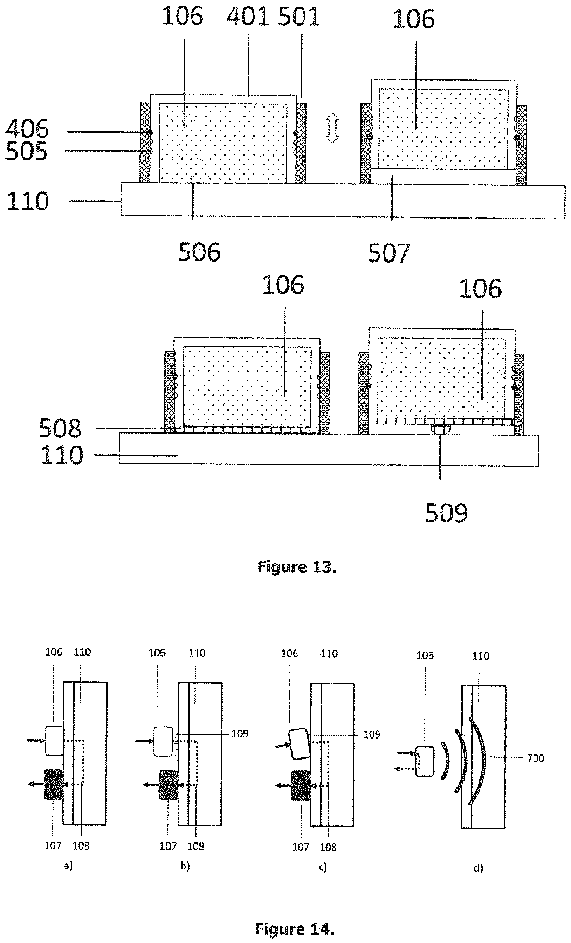

The transducer (106) is usually located in the transducer housing, and may also comprise an adaptive transducer extension (501), as shown in Figure 13, for precise positioning of the transducer (106) relative to the treated surface of the living organism, more specifically, for defining the distance between the active surface of the transducer (506) and the treated surface of the living organism, it also enables control of the discharge properties and retention of constituents generated by the appearance of a dielectric barrier discharge within a closed volume whose walls comprise the active transducer surface, the treated surface of the living organism, and the inner wall of the telescopic extension (507). The adaptive extension may also comprise a dielectric photo filter (508) that covers the active surface of the transducer for light transmission of a specific spectrum to the target treated tissue, in the case of a wide-spectrum light source usage. The adaptive extension may also comprise passive elements adjacent to the active surface of the transducer (106) and the target treated surface, which allow the same distance to be maintained for use on larger surfaces of the living organism. The transducer (106) may also include a retention element on the extension or a retention element on the transducer housing (505). If necessary, the transducer (106) may also comprise a passive element for maintaining a constant distance when used on larger surfaces (509).

The resonant electrode (107) that comes in contact with the surface of the organism is made of conductive material of arbitrary dimensions and shapes, e.g. a tube or a plate, and is coupled to the central unit (100). The resonant electrode (107) comprises an electrical circuit that allows the U_{MJ} measurement described earlier to be performed and visual or audible signaling of the establishment of a therapeutic energy resonant circuit.

In another embodiment, the present device for treatment of diseases and conditions generates impulse profiles that include impulse sequences that can be adjusted and modulated with respect to their shape that can be either square, sinusoidal, trapezoidal, triangular, sawtooth, reversed sawtooth, linear increase (rumpup), linear decrease (rumpdown), exponential growth (rumpup), exponential decay (rumpdown), even harmonics, odd harmonics, exponentially damped sine, exponentially amplified sine or modulated impulse width, with frequencies of the impulse sequences ranging from 0.1 Hz to 7,500 Hz, resonant frequency ranging from 20 to 350 Hz, amplitude modulation by an oscillating signal in the frequency range from 0.1 to 5,040 Hz, impulse amplitude ranging from 500 V to 30 kV with modulation depth ranging from 1 to 100%, wherein electromagnetic field, high-frequency currents ranging from 40 MHz to 1 GHz and sound waves ranging from 0.1 to 20,000 Hz are generated through the generation of dielectric barrier discharge in the air gap between the transducer surface and the target treated tissue surface.

The device is applied in several possible ways, as shown in Figure 14, by placing one or more transducers (106) and resonant electrodes (107) in direct contact as in Figure 14a, or immediate vicinity, as shown in Figure 14b, or combination thereof 14c with the treated surface of the organism at specific sites on the body, and specific impulse profiles designed for treating specific diseases and conditions are applied to or guide through the target treated tissue of the living organism. In the embodiment of the transducer with inductive elements (604 and 605) that are directly coupled to the block of the generator and signal profile regulator (105), a therapeutic field (700) is generated, which affects the treated organism without direct contact between the transducer active surface and the living organism, as shown in Figure 14d.

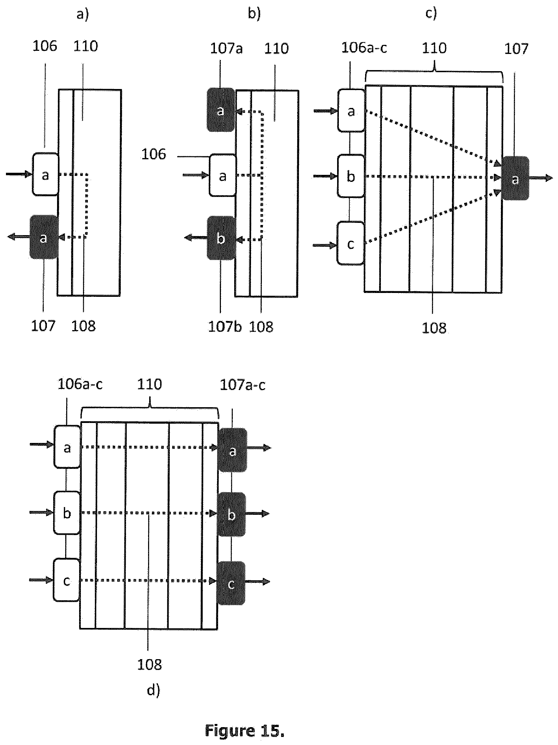

The coupling between one or more transducers (106) and one or more resonant electrodes (107) achieved through the target treated tissue by direct coupling, e.g., when applied to a wound or by capacitive coupling, e.g., when applied to the skin, it enables the establishment of a therapeutic resonant energy circuit, i.e targeted guidance of electromagnetic impulse profiles through target treated tissue, such as joint and muscle, or between target points on the tissue, such as between the reflex points at the upper and lower extremities, to stimulate certain deeper tissue structures, neurological pathways, innervation areas or energy meridians, and centers of a living organism. Some of the possible arrangements of transducers and resonant electrodes in relation to the treated tissue are shown in Figure 15. In this way, a therapeutic resonant energy pathway circuit may be applied to stimulate a particular pathway on a living organism between two (15a) or more (15b) distant points on the surface, as well as the stimulating deeper structures of a living organism with one or more pairs of transducers and resonant electrodes (15c and d), whereby in the case of multiple pairs of therapeutic electrodes a diffuse or focused effect on a specific target tissue of a living organism may be achieved by controlling the impulse profiles and starting modes (parallel or serial) of individual transducers (Figure 15d).

## Claims

1. A device for non-invasive treatment of diseases and conditions of living organisms (10) configured for simultaneous application of light, EM field, electric current, micro-vibrations, sound, and dielectric barrier discharge, suitable for establishing a therapeutic resonant energy pathway through the target treated tissue; the device comprising a central unit (100) for the production and control of high-voltage impulse profiles having an amplitude in the range from 500 V to 30.000 V, and one or more pairs of therapeutic electrodes, each pair of therapeutic electrodes comprising one transducer (106) and one resonant electrode (107), and wherein the central unit (100) is coupled to one or more of the pairs of therapeutic electrodes, and wherein the device is configured to generate sequence of high-voltage impulse profiles to be transmitted to a living organism by means of a pair of the therapeutic electrodes, wherein the transducer is configured to act as a source and the resonant electrode is configured to act as a sink**,** wherein at least one resonant electrode is suitable for the establishment of the therapeutic resonant energy path and is configured for measurement of the transmitted energy, voltage, current and impedance during treatment to control the amount of delivered energy during treatment according to the type and response of the target treated tissue,
**characterized in that** the impulse profiles are frequency- and amplitude modulated.

2. The device for treatment of diseases and conditions (10), as claimed in claim 1, **characterized in that** the central unit is designed in the form of a portable hand-held (8) or desktop device.

3. The device for treatment of diseases and conditions (10), as claimed in any of the previous claims, **characterized in that** the central unit (100) comprises housings, battery or mains power supply source (101), a management unit (102), a control unit (103), a voltage stabilizer (104), and a block comprising generator and signal profile regulator (105), which represents the source of high-voltage signal profiles and is configured for adjustment of output impulse profiles according to the type and target treated tissue response.

4. The device for treatment of diseases and conditions (10), as claimed in claim 3, **characterized in that** the block of the generator and signal profile regulator (105) comprises one or more high-voltage transformers, an energy block attached to the high-voltage transformer, wherein at least one transducer and a measuring block attached to the energy block, the resonant electrode, and the management unit are connected to the secondary side of the one or more high-voltage transformers.

5. The device for treatment of diseases and conditions (10), as claimed in claim 4, **characterized in that** the management unit (102) is configured to receive from the block of the generator and signal profile regulator (105) a voltage signal, which is generated based on the voltage and current measured between the transducer, i.e. source (106) and the resonant electrode, i.e. sink (107), wherein the voltage signal is proportional to the current energy transmitted to the therapeutic resonant energy pathway (108), wherein based on monitoring of the voltage signal, the control unit (103) is configured to determine in real time whether the device for treatment of diseases and conditions (10) is operating properly, and whether an estimated amount of energy for a particular treatment is delivered.

6. The device for treatment of diseases and conditions (10), as claimed in claims 4 or 5, **characterized in that** the voltage waveform parameters on the secondary of the high-voltage transformer of the block of the generator and signal profile regulator (105) are variable and adjustable, so that the amplitude, frequency and amplitude- and frequency modulation in time can be changed and regulated.

7. The device for treatment of diseases and conditions (10), as claimed in claims 5 or 6, **characterized in that** the waveforms of the modulation signal are square, sinusoidal, trapezoidal, triangular, sawtooth, reversed sawtooth, linear increase (rumpup), linear decrease (rumpdown), exponential growth (rumpup), exponential decay (rumpdown), even harmonics, odd harmonics, exponentially damped sine, exponentially amplified sine, modulated impulse width or combination thereof, whereby the amplitude modulation depth of the voltage signal is adjustable from 1 to 100%.

8. The device for treatment of diseases and conditions (10), as claimed in claims 5 or 6, **characterized in that** the impulse sequences on the secondary of the high-voltage transformer of the block of the generator and signal profile regulator (105) are made of impulses such as square, sinusoidal, trapezoidal, triangular, sawtooth, reversed sawtooth, linear increase (rumpup), linear decrease (rumpdown), exponential growth (rumpup), exponential decay (rumpdown), even harmonics, odd harmonics, exponentially damped or amplified sine, modulated impulse width or combination thereof, wherein the frequency impulse modulation is adjustable.

9. The device for treatment of diseases and conditions (10), as claimed in claims 5, 6, 7 or 8, **characterized in that** various therapeutic impulse profiles may be applied via the management unit (102) containing impulse sequences (impulse arrays), which can be regulated and modulated according to their waveform (sinusoidal, rectangular, trapezoidal, linear or exponential growth or decay, even or odd harmonics, damped sine), impulse sequence frequency in the range from 0.1 Hz to 7,500 Hz and resonant frequency ranging from 20 kHz to 350 kHz with frequency modulation, and a voltage amplitude of 500 V to 30,000 V with a voltage amplitude modulation by an oscillating signal in the range from 0.1 Hz to 5,040 Hz with adaptable modulation depth.

10. The device for treatment of diseases and conditions (10), as claimed in claims 5, 6, 7, 8, or 9, **characterized in that** when the transducer (106) is in the immediate vicinity of the living organism and when the device (10) is in function, the device is configured to generate a dielectric barrier discharge, generating an electromagnetic field in the frequency range from 40 MHz to 1 GHz, and micro-vibrations, i.e. sound in the frequency range from 0.1 Hz to 20 kHz, and generating in the air gap between the transducer and the surface of the treated tissue a dielectric barrier discharge and a slightly ionized cold atmospheric plasma, whereas the central unit (100) coupled to the transducer (106), which further has an inductive spark gap, generates an electromagnetic field of a wide frequency range from 1 Hz to 20 GHz.

11. The device for treatment of diseases and conditions (10), as claimed in claims 3 to 10, **characterized in that** when the device (10) is used for the tissue treatment, a transducer (106)- treated tissue (110)- resonant electrode (107) coupling may be established, whereby a therapeutic resonant energy pathway (108) may be established through the target treated tissue, whereby the block of the generator and signal profile regulator (105) measures through this coupling in real time the energy amount, voltage, current and impedance during treatment to monitor the amount of the transmitted energy and the response of the treated organism to stimulation, which depend on the administration modality, the type and condition of the target treated tissue and/or ambient conditions during application, to allow the control of the transmitted energy amount in real time by adjusting the output impulse profiles generated by the block of the generator and signal profile regulator (105).

12. The device for treatment of diseases and conditions (10), as claimed in any of the aforementioned claims, **characterized in that** the transducer (106) contains a connector (405), an insulating housing (401), a capacitive and/or inductive element (402), a dielectric barrier (404), a light source (408) configured to generate a glow discharge in a partially evacuated volume enclosed by dielectric, containing one or more gases that are, with the help of the capacitive element (402), coupled to the generator and signal profile regulator (105), excited over dielectric barrier (404) through the connector (405).

13. The device for treatment of diseases and conditions (10), as claimed in any of the aforementioned claims, **characterized in that** the transducer (106) comprises a connector (405), an insulating housing (401), a capacitive and/or inductive element (402), a dielectric barrier (404), an electronic circuit (407) with a light source (408) of LED or OLED type of light or other alternative light source in the UV, visible, or infrared portion of the spectrum, excited by separate impulse profiles generated by the management unit (102).

14. The device for treatment of diseases and conditions (10), as claimed in claims 12 or 13, **characterized in that** the dielectric barrier (404) in the transducer (106) is made of glass, ceramics or polymers.

15. The device for treatment of diseases and conditions (10), as claimed in claims 12, 13 or 14, **characterized in that** the capacitive and/or inductive element (402) is made of conductive material either as a capacitive plate (601), a disc (602), or network (603), or as an inductive disc (604), or a light-transmitting coil (605), or as a segmented coil with a spark gap (606), or as a combination thereof, and is located on the proximal side of the dielectric barrier (404).

16. The device for treatment of diseases and conditions (10), as claimed in claims 12, 13 or 14, **characterized in that** the capacitive and/or inductive element (402) is made of conductive material such as segmented coil with a spark gap (412), wherein the segmented coil with a spark gap comprises contact points for source coupling (701) and sink coupling (702), wherein the segmented coil with a spark gap (606) can be flat or conically wound, and wherein the arrangement and length of the individual segments of flat-coil or conically wound coil are of arbitrary width/thickness of wire d1, radially intersected from its center to form spaces (d2) between individual coil segments, and in order to prevent the unwanted arcing between the individual threads of the coil, the space between individual threads d3 is not less than two sizes of spaces d2, and wherein the method of intersection of the coil is defined by a circle divided into several segments in such a way that each subsequent segment, starting from the largest angle, is equal to the quotient of the previous larger angle and the value $\frac{1 + \sqrt{5}}{2}$.

17. The device for treatment of diseases and conditions (10), as claimed in claims 15 or 16, **characterized in that** inductive elements (604), (605), (606) comprise a contact point for source coupling (607) and sink coupling (608), the inductive element (604), (605), (606) being directly coupled to the block of the generator and signal profile regulator (105), wherein the operation of the device (10) is possible without a direct contact with the treated organism, but the transducer and the device (10) are positioned at a certain distance from the organism to generate a therapeutic field.

18. The device for treatment of diseases and conditions (10), as claimed in claims 14 to 17, **characterized in that** the active surface of the transducer (106) in the form of a dielectric barrier (404), which is directed or comes into contact with the treated organism, is flat (801), convex (802), concave (803) or pointed (804), or a combination thereof, to meet energy and ergonomic requirements, all depending on the type and needs of the therapeutic procedure, and is made of glass, ceramics or polymers.

19. The device for treatment of diseases and conditions (10), as claimed in claims 12 to 18, **characterized in that** the transducer (106) comprises adaptive extensions (501) for precise positioning of the transducer (106) relative to the treated surface of the living organism, furthermore it contains a telescopic extension (507) with an inner wall whose sliding can precisely define the distance between the active surface of the transducer (dielectric barrier) and the treated surface of the living organism, allowing during the operation of the device (10) the control of the dielectric barrier discharge properties and retention of constituents generated by the appearance of a dielectric barrier discharge within a closed and clearly defined volume whose walls consist of the the active transducer surface (106), the treated surface of the living organism, and the inner wall of the telescopic extension (507).

20. The device for treatment of diseases and conditions (10), as claimed in claim 19, **characterized in that** the transducer (106) contains adaptive extensions (501) including a dielectric photo filter (508) that covers the active surface of the transducer (106) by transmitting a specific spectrum of light onto the target treated tissue.

21. The device for treatment of diseases and conditions (10), as claimed in claims 19 or 20, **characterized in that** the transducer (106) comprises adaptive extensions (501) containing one or more passive elements (509) for maintaining a constant distance between the dielectric barrier discharge and the surface of the target treated tissue.

22. The device for treatment of diseases and conditions (10), as claimed in any of the aforementioned claims, **characterized in that** the resonant electrode (107) is configured to come in contact with the surface of the organism, the latter being made of conductive material of arbitrary dimensions and shapes, and coupled to the central unit (100), wherein the resonant electrode (107) comprises an electronic circuit configured to enable voltage measurements and visual or audible signaling to establish a therapeutic resonant energy circuit (108).

23. The device for treatment of diseases and conditions (10), as claimed in any of the aforementioned claims, **characterized in that** it is confgured to generate impulse profiles that include impulse sequences that can be adapted and modulated with respect to their shape, which may be either sinusoidal, rectangular, triangular, or sawtooth, trapezoidal, linear or exponential growth or decay, even or odd harmonics, exponentially damped or amplified sine with frequency of impulse sequences ranging from 0.1 Hz to 7,500 Hz, the frequency of modulated impulses ranging from 20 to 350 Hz, wherein the modulation frequency of the modulation signal amplitude by an oscillating signal is in the range from 0.1 to 5,040 Hz, wherein the impulse amplitude is in the range of about 500 V to 30 kV, and wherein the electromagnetic field and high-frequency currents ranging from 40 MHz to 1 GHz and sound waves ranging from 0.1 to 20,000 Hz are generated through the generation of a dielectric barrier in the air gap between the transducer surface and the surface of the target treated tissue.

24. The device for treatment of diseases and conditions (10), as claimed in any of the aforementioned claims, **characterized in that** it is configured to generate impulse profiles that include impulse sequences that can be adapted and modulated considering the impulse sequence frequency ranging from 0.1 to 7,500 Hz, the frequency of modulated impulses ranging from 20 to 350 kHz and the frequency of the modulation signal in the range from 0.1 to 5,040 Hz.

25. The device for treatment of diseases and conditions (10), as claimed in any of the aforementioned claims, **characterized in that** the impulse sequence frequency is 0.72 Hz or any harmonic thereof including 1.44 or 2.16 or 2.88 or 3.6 or 4.32 or 7.2 or 14.4 or 21.6 or 28.8 or 36 or 43.2 or 50.4 or 57.6 or 64.8 or 72 or 144 or 216 or 288 or 360 or 432 or 504 or 576 or 648 or 720 or 792 or 864 or 936 or 1,008 or 1,080 or 1,152 or 1,224 or 1,296 or 1,368 or 1,440 or 2,160 or 2,880 or 3,600 or 4,320 or 5,040 or 5,760 or 6,480 or 7,200 or 7,344 Hz or sweep over the specified frequency range.

26. The device for treatment of diseases and conditions (10), as claimed in any of the aforementioned claims, **characterized in that** the frequency of the modulated impulses is 21.6 or 28.8 or 36 or 43.2 or 50.4 or 57.6 or 64.8 or 72 or 79.2 or 86.4 or 93.6 or 100.8 or 108 or 115.2 or 122.4 or 129.6 or 136.8 or 144 or 151.2 or 158.4 or 165.6 or 172.8 or 180 or 187.2 or 194.4 or 201.6 or 208.8 or 216 or 223.2 or 230.4 or 237.6 or 244.8 or 252 or 259.2 or 266.4 or 273.6 or 280.8 or 288 or 295.2 or 302.4 or 309.6 or 316.8 or 324 or 331.2 or 338.4 or 345.6 kHz or sweep over the specified frequency range.

27. The device for treatment of diseases and conditions (10), as claimed in any of the aforementioned claims, **characterized in that** the frequency of the modulation signal is 0.72 Hz or any of its harmonics including 1.44 or 2.16 or 2.88 or 3.6 or 4.32 or 7.2 or 14.4 or 21.6 or 28.8 or 36 or 43.2 or 50.4 or 57.6 or 64.8 or 72 or 144 or 216 or 288 or 360 or 432 or 504 or 576 or 648 or 720 or 792 or 864 or 936 or 1,008 or 1,080 or 1,152 or 1,224 or 1,296 or 1,368 or 1,440 or 2,160 or 2,880 or 3,600 or 4,320 or 5,040 Hz or sweep over the mentioned frequency range.

28. The device for treatment of diseases and conditions (10), as claimed in claims 25, 26 or 27, **characterized in that** the voltage on the secondary of the high-voltage transformer of the block of the generator and the signal profile regulator (105) is generated as an impulse sequence whose waveform is either sinusoidal, rectangular, triangular or sawtooth, trapezoidal, linear or exponential growth or decay, even or odd harmonic, exponentially damped or amplified sine, the impulse sequences being modulated by sinusoidal, rectangular, trapezoidal, linear or exponential growth or decay, even or odd harmonics, damped sinusoidal waveforms of the voltage managing signal (modulation signal) of frequency ranging from 0.72 to 5,040 Hz, with a modulation amplitude depth ranging from 1 to 100%.

## Patentansprüche

1. Vorrichtung zur nichtinvasiven Behandlung von Krankheiten und Zuständen lebender Organismen (10), konfiguriert zur gleichzeitigen Anwendung von Licht, elektromagnetischem Feld, elektrischem Strom, Mikrovibrationen, Schall und dielektrischer Barrierenentladung, geeignet zur Ausbildung eines therapeutischen Resonanzenergiepfades durch das zu behandelnde Zielgewebe, umfassend eine Zentraleinheit (100) zur Erzeugung und Steuerung von Hochspannungsimpulsprofilen mit einer Amplitude im Bereich von 500 V bis 30 000 V sowie ein oder mehrere Paare therapeutischer Elektroden, wobei jedes Paar therapeutischer Elektroden einen Wandler (106) und eine Resonanzelektrode (107) umfasst, wobei die Zentraleinheit (100) mit einem oder mehreren Paaren therapeutischer Elektroden gekoppelt ist, und wobei die Vorrichtung dazu konfiguriert ist, eine Folge von Hochspannungsimpulsprofilen über ein Paar therapeutischer Elektroden auf einen lebenden Organismus zu übertragen, wobei der Wandler als Quelle und die Resonanzelektrode als Senke ausgebildet ist, wobei wenigstens eine Resonanzelektrode zum Aufbau des therapeutischen Resonanzenergiepfades geeignet und zur Messung der während der Behandlung übertragenen Energie, Spannung, Strom und Impedanz konfiguriert ist, um die Menge der während der Behandlung übertragenen Energie entsprechend der Art des zu behandelnden Zielgewebes und dessen Reaktion auf die Behandlung zu steuern, **dadurch gekennzeichnet, dass** die Impulsprofile frequenz- und amplitudenmoduliert sind.

2. Vorrichtung zur Behandlung von Krankheiten und Zuständen (10) nach Anspruch 1, **dadurch gekennzeichnet, dass** die Zentraleinheit als tragbares Handgerät (8) oder als Tischgerät konfiguriert ist.

3. Vorrichtung zur Behandlung von Krankheiten und Zuständen (10) nach einem der vorhergehenden Ansprüche, **dadurch gekennzeichnet, dass** die Zentraleinheit (100) ein Gehäuse, eine Batteriestromversorgungsquelle oder eine Netzstromversorgungsquelle (101), eine Steuereinheit (102), eine Regelungseinheit (103), einen Spannungsstabilisator (104) sowie eine Baugruppe aus Generator und Signalprofilregler (105) umfasst, wobei die Baugruppe aus Generator und Signalprofilregler (105) die Quelle der Hochspannungssignalprofile bildet und zur Anpassung der Ausgangsimpulsprofile entsprechend der Art und der Reaktion des zu behandelnden Zielgewebes konfiguriert ist.

4. Vorrichtung zur Behandlung von Krankheiten und Zuständen (10) nach Anspruch 3, **dadurch gekennzeichnet, dass** die Baugruppe aus Generator und Signalprofilregler (105) einen oder mehrere Hochspannungstransformatoren sowie eine mit dem Hochspannungstransformator verbundene Energieeinheit umfasst, wobei wenigstens ein Wandler sowie eine mit der Energieeinheit, der Resonanzelektrode sowie mit der Steuereinheit verbundene Messeinheit mit der Sekundärseite eines oder mehrerer Hochspannungstransformatoren verbunden sind.

5. Vorrichtung zur Behandlung von Krankheiten und Zuständen (10) nach Anspruch 4, **dadurch gekennzeichnet, dass** die Steuereinheit (102) dazu konfiguriert ist, von der Baugruppe aus Generator und Signalprofilregler (105) ein Spannungssignal zu empfangen, das auf der zwischen dem Wandler, d. h. der Quelle (106), und der Resonanzelektrode, d. h. der Senke (107), gemessenen Spannung und Strom basiert, wobei das Spannungssignal proportional zur momentan über den therapeutischen resonanten Energiepfad übertragenen Energie ist, und wobei die Regelungseinheit (103) auf der Grundlage der Überwachung des Spannungssignals dazu konfiguriert ist, in Echtzeit festzustellen, ob die Vorrichtung zur Behandlung von Krankheiten und Zuständen (10) ordnungsgemäß arbeitet, und ob eine geschätzte Energiemenge für eine bestimmte Behandlung übertragen wird.

6. Vorrichtung zur Behandlung von Krankheiten und Zuständen (10) nach Anspruch 4 oder 5, **dadurch gekennzeichnet, dass** die Parameter der Spannungswellenform an der Sekundärseite des Hochspannungstransformators der Baugruppe aus Generator und Signalprofilregler (105) variabel und einstellbar sind, sodass Amplitude, Frequenz sowie Amplituden- und Frequenzmodulation zeitlich verändert und eingestellt werden können.

7. Vorrichtung zur Behandlung von Krankheiten und Zuständen (10) nach Anspruch 5 oder 6, **dadurch gekennzeichnet, dass** die Wellenformen des Modulationssignals rechteckförmig, sinusförmig, trapezförmig, dreieckförmig, sägezahnförmig, umgekehrt sägezahnförmig, linear ansteigend (Ramp-up), linear abfallend (Ramp-down), exponentiell ansteigend (Ramp-up), exponentiell abfallend (Ramp-down), gerade Harmonische, ungerade Harmonische, exponentiell gedämpfter Sinus, exponentiell verstärkter Sinus, impulsbreitenmoduliert oder Kombinationen hiervon sind, wobei die Modulationstiefe der Amplitude des Spannungssignals von 1 % bis 100 % einstellbar ist.

8. Vorrichtung zur Behandlung von Krankheiten und Zuständen (10) nach Anspruch 5 oder 6, **dadurch gekennzeichnet, dass** die Impulsfolgen an der Sekundärseite des Hochspannungstransformators der Baugruppe aus Generator und Signalprofilregler (105) aus rechteckförmigen, sinusförmigen, trapezförmigen, dreieckförmigen, sägezahnförmigen, umgekehrt sägezahnförmigen, linear ansteigenden (Ramp-up), linear abfallenden (Ramp-down), exponentiell ansteigenden (Ramp-up), exponentiell abfallenden (Ramp-down) Imspulsen, Impulsen mit geraden Harmonischen, Impulsen mit ungeraden Harmonischen, exponentiell gedämpften oder exponentiell verstärkten Sinusimpulsen, pulsweitenmodulierten Impulsen oder Kombinationen hiervon bestehen, wobei die Impulsfrequenzmodulation einstellbar ist.

9. Vorrichtung zur Behandlung von Krankheiten und Zuständen (10) nach einem der Ansprüche 5, 6, 7 oder 8, **dadurch gekennzeichnet, dass** über die Steuereinheit (102) verschiedene therapeutische Impulsprofile angewendet werden können, die Impulsfolgen (Impulsarrays) enthalten, wobei diese entsprechend ihrer Wellenform (sinusförmig, rechteckförmig, trapezförmig, linear oder exponentiell ansteigend oder abfallend, gerade oder ungerade Harmonische, gedämpfter Sinus), der Frequenz der Impulsfolgen im Bereich von 0,1 Hz bis 7 500 Hz, der Resonanzfrequenz im Bereich von 20 kHz bis 350 kHz mit Frequenzmodulation sowie der Spannungsamplitude von 500 V bis 30 000 V mit einer Spannungsamplitudenmodulation durch ein oszillierendes Signal im Frequenzbereich von 0,1 Hz bis 5 040 Hz und anpassbarer Modulationstiefe geregelt und moduliert werden können.

10. Vorrichtung zur Behandlung von Krankheiten und Zuständen (10) nach einem der Ansprüche 5, 6, 7, 8 oder 9, **dadurch gekennzeichnet, dass** die Vorrichtung, wenn sich der Wandler (106) in unmittelbarer Nähe des lebenden Organismus befindet und die Vorrichtung (10) in Betrieb ist, dazu konfiguriert ist, eine dielektrische Barrierenentladung zu erzeugen, wobei ein elektromagnetisches Feld im Frequenzbereich von 40 MHz bis 1 GHz und Mikrovibrationen, d. h. Schall im Frequenzbereich von 0,1 Hz bis 20 kHz erzeugt werden, und wobei im Luftspalt zwischen dem Wandler und der Oberfläche des zu behandelnden Zielgewebes eine dielektrische Barrierenentladung sowie ein schwach ionisiertes kaltes Atmosphärenplasma erzeugt werden, während der mit der Zentraleinheit (100) gekoppelte Wandler (106), der ferner über eine induktive Funkenstrecke verfügt, ein elektromagnetisches Feld in einem breiten Frequenzbereich von 1 Hz bis 20 GHz erzeugt.

11. Vorrichtung zur Behandlung von Krankheiten und Zuständen (10) nach einem der Ansprüche 3 bis 10, **dadurch gekennzeichnet, dass** bei Anwendung der Vorrichtung (10) zur Behandlung von Gewebe eine Kopplung Wandler (106) - behandeltes Gewebe (110) - Resonanzelektrode (107) hergestellt werden kann, wobei durch das zu behandelnde Zielgewebe ein therapeutischer resonanter Energiepfad (108) aufgebaut werden kann, wobei die Baugruppe aus Generator und Signalprofilregler (105) über diese Kopplung in Echtzeit die Energiemenge, die Spannung, den Strom und die Impedanz während der Behandlung misst, wodurch die übertragene Energiemenge sowie die Reaktion des behandelten Organismus auf die applizierte Stimulation überwacht werden, wobei diese von der Anwendungsmodalität, der Art und dem Zustand des zu behandelnden Zielgewebes und/oder den Umgebungsbedingungen während der Anwendung abhängen, und wodurch die Regelung der übertragenen Energiemenge in Echtzeit durch Anpassung der von der Baugruppe aus Generator und Signalprofilregler (105) erzeugten Ausgangsimpulsprofile ermöglicht wird.

12. Vorrichtung zur Behandlung von Krankheiten und Zuständen (10) nach einem der vorhergehenden Ansprüche, **dadurch gekennzeichnet, dass** der Wandler (106) einen Steckverbinder (405), ein Isoliergehäuse (401), ein kapazitives und/oder induktives Element (402), eine dielektrische Barriere (404) sowie eine Lichtquelle (408) umfasst, und dazu konfiguriert ist, eine Glimmentladung in einem teilweise evakuierten, durch das Dielektrikum begrenzten Volumen zu erzeugen, in dem sich ein oder mehrere Gase befinden, die mittels des kapazitiven Elements (402) mit der Baugruppe aus Generator und Signalprofilregler (105) gekoppelt sind und über die dielektrische Barriere (404) mittels des Verbinders (405) angeregt werden.

13. Vorrichtung zur Behandlung von Krankheiten und Zuständen (10) nach einem der vorhergehenden Ansprüche **dadurch gekennzeichnet, dass** der Wandler (106) einen Steckverbinder (405), ein Isoliergehäuse (401), ein kapazitives und/oder induktives Element (402), eine dielektrische Barriere (404) sowie eine elektronische Schaltung (407) mit einer LED- oder OLED-Lichtquelle (408) oder einer anderen Lichtquelle zur Emission von ultraviolettem, sichtbarem oder infrarotem Licht umfasst, wobei die Lichtquelle durch getrennte Impulsprofile angesteuert wird, die von der Steuereinheit (102) erzeugt werden.

14. Vorrichtung zur Behandlung von Krankheiten und Zuständen (10) nach Anspruch 12 oder 13, **dadurch gekennzeichnet, dass** die dielektrische Barriere (404) im Wandler (106) aus Glas, Keramik oder Polymeren besteht.

15. Vorrichtung zur Behandlung von Krankheiten und Zuständen (10) nach Anspruch 12, 13 oder 14, **dadurch gekennzeichnet, dass** das kapazitive und/oder induktive Element (402) aus elektrisch leitfähigem Material besteht und als kapazitive Platte (601), Scheibe (602) oder Netz (603), oder als induktive Scheibe (604), oder als lichtdurchlässige Spule (605), oder als segmentierte Spule mit einer Funkenstrecke (606) oder als Kombination derselben ausgebildet ist, wobei das kapazitive und/oder induktive Element (402) auf der proximalen Seite der dielektrischen Barriere (404) angeordnet ist.

16. Vorrichtung zur Behandlung von Krankheiten und Zuständen (10) nach Anspruch 12, 13 oder 14, **dadurch gekennzeichnet, dass** das kapazitive und/oder induktive Element (402) aus elektrisch leitfähigem Material besteht und als segmentierte Spule mit einer Funkenstrecke (412) ausgebildet ist, wobei die segmentierte Spule mit einer Funkenstrecke Kontaktstellen für die Quellenkopplung (701) und die Senkenkopplung (702) umfasst, wobei die segmentierte Spule mit einer Funkenstrecke (606) flach oder konisch gewickelt sein kann, wobei die Anordnung und die Länge der einzelnen Segmente der flach oder konisch gewickelten Spule bei beliebiger Drahtbreite bzw. Drahtdicke d1 frei wählbar sind und wobei die Spule von ihrem Zentrum aus radial eingeschnitten ist, sodass Zwischenräume (d2) zwischen den einzelnen Spulensegmenten gebildet werden, wobei zur Vermeidung einer unerwünschten Funkenbildung zwischen den einzelnen Windungen der Spule der Abstand d3 zwischen den einzelnen Windungen nicht kleiner als das Zweifache des Abstandes d2 ist und wobei die Art der Segmentierung der Spule durch einen Kreis definiert ist, der in mehrere Segmente unterteilt ist, wobei jedes nachfolgende Segment, beginnend mit dem größten Winkel, dem Quotienten aus dem jeweils vorhergehenden größeren Winkel und dem Wert a $\frac{1 + \sqrt{5}}{2}$ entspricht.

17. Vorrichtung zur Behandlung von Krankheiten und Zuständen (10) nach Anspruch 15 oder 16, **dadurch gekennzeichnet, dass** die induktiven Elemente (604), (605) und (606) jeweils einen Kontaktpunkt für die Quellenkopplung (607) und die Senkenkopplung (608) umfassen, wobei das induktive Element (604), (605), (606) unmittelbar mit der Baugruppe aus Generator und Signalprofilregler (105) gekoppelt ist, und wobei der Betrieb der Vorrichtung (10) ohne direkten Kontakt mit dem behandelten Organismus möglich ist, wobei der Wandler und die Vorrichtung (10) in einem bestimmten Abstand vom Organismus angeordnet sind, um ein therapeutisches Feld zu erzeugen.

18. Vorrichtung zur Behandlung von Krankheiten und Zuständen (10) nach einem der Ansprüche 14 bis 17, **dadurch gekennzeichnet, dass** die aktive Oberfläche des Wandlers (106) in Form einer dielektrischen Barriere (404), welche auf den behandelten Organismus gerichtet ist oder mit diesem in Kontakt kommt, eben (801), konvex (802), konkav (803), spitz (804) oder als Kombination hiervon ausgebildet ist, um den energetischen und ergonomischen Anforderungen entsprechend der Art und den Erfordernissen des therapeutischen Verfahrens gerecht zu werden, wobei die aktive Oberfläche aus Glas, Keramik oder Polymeren besteht.

19. Vorrichtung zur Behandlung von Krankheiten und Zuständen (10) nach einem der Ansprüche 12 bis 18, **dadurch gekennzeichnet, dass** der Wandler (106) Anpassungselemente (501) zur präzisen Positionierung des Wandlers (106) relativ zur behandelten Oberfläche des lebenden Organismus umfasst, wobei ferner eine teleskopische Verlängerung (507) mit einer Innenwand vorgesehen ist, deren Verschiebung den Abstand zwischen der aktiven Oberfläche des Wandlers (dielektrische Barriere) und der behandelten Oberfläche des lebenden Organismus präzise definiert, wodurch während des Betriebs der Vorrichtung (10) die Eigenschaften der dielektrischen Barrierenentladung sowie das Zurückhalten der durch die Entstehung der dielektrischen Barrierenentladung gebildeten Bestandteile innerhalb eines geschlossenen und eindeutig definierten Volumens gesteuert werden, dessen Begrenzungsflächen durch die aktive Oberfläche des Wandlers (106), die behandelte Oberfläche des lebenden Organismus sowie die Innenwand der teleskopischen Verlängerung (507) gebildet werden.

20. Vorrichtung zur Behandlung von Krankheiten und Zuständen (10) nach Anspruch 19, **dadurch gekennzeichnet, dass** der Wandler (106) Anpassungselemente (501) umfasst, welche einen dielektrischen Photofilter (508) enthalten, der die aktive Oberfläche des Wandlers (106) bedeckt und ein bestimmtes Lichtspektrum zum zu behandelnden Zielgewebe durchlässt.

21. Vorrichtung zur Behandlung von Krankheiten und Zuständen (10) nach Anspruch 19 oder 20, **dadurch gekennzeichnet, dass** der Wandler (106) Anpassungselemente (501) umfasst, welche ein oder mehrere passive Elemente (509) enthalten, um einen konstanten Abstand zwischen der dielektrischen Barrierenentladung und der Oberfläche des zu behandelnden Zielgewebes aufrechtzuerhalten.

22. Vorrichtung zur Behandlung von Krankheiten und Zuständen (10) nach einem der vorhergehenden Ansprüche, **dadurch gekennzeichnet, dass** die Resonanzelektrode (107) dazu konfiguriert ist, mit der Oberfläche des Organismus in Kontakt zu kommen, wobei diese aus elektrisch leitfähigem Material beliebiger Abmessungen und Form besteht und mit der Zentraleinheit (100) gekoppelt ist, wobei die Resonanzelektrode (107) eine elektronische Schaltung umfasst, die zur Durchführung von Spannungsmessungen sowie zur visuellen oder akustischen Signalisierung konfiguriert ist, um einen therapeutischen Resonanzenergiekreis (108) aufzubauen.

23. Vorrichtung zur Behandlung von Krankheiten und Zuständen (10) nach einem der vorhergehenden Patentansprüche, **dadurch gekennzeichnet, dass** sie zur Erzeugung von Impulsprofilen konfiguriert ist, die Impulsfolgen umfassen, welche hinsichtlich ihrer Form anpassbar und modulierbar sind, wobei die Form sinusförmig, rechteckförmig, dreieckförmig, sägezahnförmig, trapezförmig, linear ansteigend, linear abfallend, exponentiell ansteigend, exponentiell abfallend, gerade Harmonische, ungerade Harmonische, exponentiell gedämpfter oder exponentiell verstärkter Sinus sein kann, wobei die Frequenz der Impulsfolgen im Bereich von 0,1 Hz bis 7 500 Hz liegt, wobei die Frequenz der modulierten Impulse im Bereich von 20 kHz bis 350 kHz liegt, wobei die Frequenz der Amplitudenmodulation des Modulationssignals durch ein Oszillatorsignal im Frequenzbereich von 0,1 Hz bis 5 040 Hz liegt, wobei die Impulsamplitude im Bereich von etwa 500 V bis 30 kV liegt und wobei ein elektromagnetisches Feld sowie Hochfrequenzströme im Bereich von 40 MHz bis 1 GHz und Schallwellen im Bereich von 0,1 Hz bis 20 000 Hz durch die Erzeugung einer dielektrischen Barriere im Luftzwischenraum zwischen der Oberfläche des Transduktors und der Oberfläche des zu behandelnden Zielgewebes erzeugt werden.

24. Vorrichtung zur Behandlung von Krankheiten und Zuständen (10) nach einem der vorhergehenden Patentansprüche, **dadurch gekennzeichnet, dass** sie zur Erzeugung von Impulsprofilen konfiguriert ist, die Impulsfolgen umfassen, welche hinsichtlich der Frequenz der Impulsfolgen im Bereich von 0,1 Hz bis 7 500 Hz, der Frequenz der modulierten Impulse im Bereich von 20 kHz bis 350 kHz sowie der Frequenz des Modulationssignals im Bereich von 0,1 Hz bis 5 040 Hz anpassbar und modulierbar sind.

25. Vorrichtung zur Behandlung von Krankheiten und Zuständen (10) nach einem der vorhergehenden Patentansprüche, **dadurch gekennzeichnet, dass** die Frequenz der Impulsfolge 0,72 Hz oder eine Harmonische davon ist, insbesondere 1,44 Hz, 2,16 Hz, 2,88 Hz, 3,6 Hz, 4,32 Hz, 7,2 Hz, 14,4 Hz, 21,6 Hz, 28,8 Hz, 36 Hz, 43,2 Hz, 50,4 Hz, 57,6 Hz, 64,8 Hz, 72 Hz, 144 Hz, 216 Hz, 288 Hz, 360 Hz, 432 Hz, 504 Hz, 576 Hz, 648 Hz, 720 Hz, 792 Hz, 864 Hz, 936 Hz, 1 008 Hz, 1 080 Hz, 1 152 Hz, 1 224 Hz, 1 296 Hz, 1 368 Hz, 1 440 Hz, 2 160 Hz, 2 880 Hz, 3 600 Hz, 4 320 Hz, 5 040 Hz, 5 760 Hz, 6 480 Hz, 7 200 Hz oder 7 344 Hz oder ein Sweep über diesen Frequenzbereich erfolgt.

26. Vorrichtung zur Behandlung von Krankheiten und Zuständen (10) nach einem der vorhergehenden Patentansprüche, **dadurch gekennzeichnet, dass** die Frequenz der modulierten Impulse 21,6 kHz oder 28,8 kHz oder 36 kHz oder 43,2 kHz oder 50,4 kHz oder 57,6 kHz oder 64,8 kHz oder 72 kHz oder 79,2 kHz oder 86,4 kHz oder 93,6 kHz oder 100,8 kHz oder 108 kHz oder 115,2 kHz oder 122,4 kHz oder 129,6 kHz oder 136,8 kHz oder 144 kHz oder 151,2 kHz oder 158,4 kHz oder 165,6 kHz oder 172,8 kHz oder 180 kHz oder 187,2 kHz oder 194,4 kHz oder 201,6 kHz oder 208,8 kHz oder 216 kHz oder 223,2 kHz oder 230,4 kHz oder 237,6 kHz oder 244,8 kHz oder 252 kHz oder 259,2 kHz oder 266,4 kHz oder 273,6 kHz oder 280,8 kHz oder 288 kHz oder 295,2 kHz oder 302,4 kHz oder 309,6 kHz oder 316,8 kHz oder 324 kHz oder 331,2 kHz oder 338,4 kHz oder 345,6 kHz ist oder ein Sweep über diesen Frequenzbereich erfolgt.

27. Vorrichtung zur Behandlung von Krankheiten und Zuständen (10) nach einem der vorhergehenden Patentansprüche, **dadurch gekennzeichnet, dass** die Frequenz des Modulationssignals 0,72 Hz oder eine Harmonische davon ist, insbesondere 1,44 Hz, 2,16 Hz, 2,88 Hz, 3,6 Hz, 4,32 Hz, 7,2 Hz, 14,4 Hz, 21,6 Hz, 28,8 Hz, 36 Hz, 43,2 Hz, 50,4 Hz, 57,6 Hz, 64,8 Hz, 72 Hz, 144 Hz, 216 Hz, 288 Hz, 360 Hz, 432 Hz, 504 Hz, 576 Hz, 648 Hz, 720 Hz, 792 Hz, 864 Hz, 936 Hz, 1 008 Hz, 1 080 Hz, 1 152 Hz, 1 224 Hz, 1 296 Hz, 1 368 Hz, 1 440 Hz, 2 160 Hz, 2 880 Hz, 3 600 Hz, 4 320 Hz, 5 040 Hz oder ein Sweep über diesen Frequenzbereich erfolgt.

28. Vorrichtung zur Behandlung von Krankheiten und Zuständen (10) nach einem der Patentansprüche 25, 26 oder 27, **dadurch gekennzeichnet, dass** die Spannung an der Sekundärseite des Hochspannungstransformators der Baugruppe aus Generator und Signalprofilregler (105) als Impulsfolge erzeugt wird, deren Wellenform sinusförmig, rechteckförmig, dreieckförmig, sägezahnförmig, trapezförmig, linear oder exponentiell ansteigend oder abfallend, als gerade oder ungerade Harmonische sowie exponentiell gedämpfter oder exponentiell verstärkter Sinus ist, wobei die Impulsfolgen mit sinusförmigen, rechteckförmigen, trapezförmigen, linear oder exponentiell ansteigenden oder abfallenden, geraden oder ungeraden Harmonischen sowie mit gedämpft sinusförmigen Wellenformen des Steuerspannungssignals (Modulationssignals) mit einer Frequenz von 0,72 Hz bis 5 040 Hz moduliert sind, wobei die Modulationstiefe der Amplitudenmodulation 1 % bis 100 % beträgt.

## Revendications

1. Dispositif destiné au traitement non invasif de maladies et d'états pathologiques d'organismes vivants (10), configuré pour appliquer simultanément de la lumière, un champ électromagnétique (EM), un courant électrique, des micro-vibrations, des sons et une décharge à barrière diélectrique, apte à établir une voie énergétique résonante thérapeutique à travers le tissu cible traité;
ledit dispositif comprenant :
une unité centrale (100) destinée à produire et à commander des profils d'impulsions à haute tension ayant une amplitude comprise entre 500 V et 30 000 V, et
une ou plusieurs paires d'électrodes thérapeutiques, chaque paire d'électrodes thérapeutiques comprenant un transducteur (106) et une électrode résonante (107),
l'unité centrale (100) étant couplée à une ou plusieurs desdites paires d'électrodes thérapeutiques,
le dispositif étant configuré pour générer une séquence de profils d'impulsions à haute tension destinée à être transmise à un organisme vivant au moyen d'une desdites paires d'électrodes thérapeutiques,
le transducteur étant configuré pour agir en tant que source et l'électrode résonante étant configurée pour agir en tant que récepteur,
au moins une électrode résonante (107) étant apte à établir ladite voie énergétique résonante thérapeutique et étant configurée pour mesurer, pendant le traitement, l'énergie transmise, la tension, le courant et l'impédance, afin de commander la quantité d'énergie délivrée pendant le traitement en fonction du type de tissu cible traité et de la réponse de celui-ci,
**caractérisé en ce que** les profils d'impulsions sont modulés en fréquence et en amplitude.

2. Dispositif de traitement de maladies et d'états pathologiques (10) selon la revendication 1, **caractérisé en ce que** l'unité centrale est réalisée sous la forme d'un dispositif portatif à main (8) ou d'un dispositif de table.

3. Dispositif de traitement de maladies et d'états pathologiques (10) selon l'une quelconque des revendications précédentes, **caractérisé en ce que** l'unité centrale (100) comprend un boîtier, une source d'alimentation (101) constituée d'une batterie ou d'une alimentation sur secteur, une unité de gestion (102), une unité de commande (103), un stabilisateur de tension (104) et un bloc générateur et régulateur de profils de signaux (105), ledit bloc constituant la source des profils de signaux à haute tension et étant configuré pour ajuster les profils d'impulsions de sortie en fonction du type de tissu cible traité et de la réponse de celui-ci.

4. Dispositif de traitement de maladies et d'états pathologiques (10) selon la revendication 3, **caractérisé en ce que** le bloc générateur et régulateur de profils de signaux (105) comprend un ou plusieurs transformateurs haute tension, un bloc d'énergie raccordé audit ou auxdits transformateurs haute tension, au moins un transducteur, ainsi qu'un bloc de mesure raccordé au bloc d'énergie, l'électrode résonante et l'unité de gestion étant connectés au secondaire dudit ou desdits transformateurs haute tension.

5. Dispositif de traitement de maladies et d'états pathologiques (10) selon la revendication 4, **caractérisé en ce que** l'unité de gestion (102) est configurée pour recevoir, en provenance du bloc générateur et régulateur de profils de signaux (105), un signal de tension généré sur la base de la tension et du courant mesurés entre le transducteur, constituant la source (106), et l'électrode résonante, constituant le récepteur (107), ledit signal de tension étant proportionnel à l'énergie instantanée transmise à la voie énergétique résonante thérapeutique (108), l'unité de commande (103) étant configurée, sur la base de la surveillance dudit signal de tension, pour déterminer en temps réel si le dispositif de traitement de maladies et d'états pathologiques (10) fonctionne correctement et si la quantité estimée d'énergie requise pour un traitement donné est délivrée.

6. Dispositif de traitement de maladies et d'états pathologiques (10) selon les revendications 4 ou 5, **caractérisé en ce que** les paramètres de la forme d'onde de la tension au secondaire du transformateur haute tension du bloc générateur et régulateur de profils de signaux (105) sont variables et réglables, de manière à permettre la modification et la régulation de l'amplitude, de la fréquence ainsi que de la modulation d'amplitude et de fréquence au cours du temps.

7. Dispositif de traitement de maladies et d'états pathologiques (10) selon les revendications 5 ou 6, **caractérisé en ce que** les formes d'onde du signal de modulation sont des formes carrées, sinusoïdales, trapézoïdales, triangulaires, en dents de scie, en dents de scie inversées, à rampe linéaire croissante (ramp-up), à rampe linéaire décroissante (ramp-down), à croissance exponentielle (ramp-up), à décroissance exponentielle (ramp-down), des harmoniques paires, des harmoniques impaires, une sinusoïde amortie exponentiellement, une sinusoïde amplifiée exponentiellement, une modulation de la largeur d'impulsion ou une combinaison de celles-ci, la profondeur de modulation d'amplitude du signal de tension étant réglable de 1 à 100 %.

8. Dispositif de traitement de maladies et d'états pathologiques (10) selon les revendications 5 ou 6, **caractérisé en ce que** les séquences d'impulsions au secondaire du transformateur haute tension du bloc générateur et régulateur de profils de signaux (105) sont constituées d'impulsions présentant des formes telles que carrée, sinusoïdale, trapézoïdale, triangulaire, en dents de scie, en dents de scie inversées, à augmentation linéaire (ramp-up), à diminution linéaire (ramp-down), à croissance exponentielle (ramp-up), à décroissance exponentielle (ramp-down), à harmoniques paires, à harmoniques impaires, sinusoïdales amorties ou amplifiées exponentiellement, à largeur d'impulsion modulée, ou une combinaison de celles-ci, la modulation de fréquence des impulsions étant réglable.

9. Dispositif de traitement de maladies et d'états pathologiques (10) selon les revendications 5, 6, 7 ou 8, **caractérisé en ce que** différents profils d'impulsions thérapeutiques peuvent être appliqués par l'intermédiaire de l'unité de gestion (102) contenant des séquences d'impulsions (trains d'impulsions), lesquelles peuvent être régulées et modulées selon leur forme d'onde (sinusoïdale, rectangulaire, trapézoïdale, à croissance ou décroissance linéaire ou exponentielle, à harmoniques paires ou impaires, sinusoïdale amortie), la fréquence des séquences d'impulsions étant comprise entre 0,1 Hz et 7 500 Hz et la fréquence de résonance étant comprise entre 20 kHz et 350 kHz, avec une modulation de fréquence et une amplitude de tension comprise entre 500 V et 30 000 V, l'amplitude de tension étant modulée par un signal oscillant dans une plage comprise entre 0,1 Hz et 5 040 Hz avec une profondeur de modulation adaptable.

10. Dispositif de traitement de maladies et d'états pathologiques (10) selon les revendications 5, 6, 7, 8 ou 9, **caractérisé en ce que**, lorsque le transducteur (106) est situé à proximité immédiate d'un organisme vivant et lorsque le dispositif (10) est en fonctionnement, le dispositif est configuré pour générer une décharge à barrière diélectrique, générant un champ électromagnétique dans une plage de fréquences comprise entre 40 MHz et 1 GHz, ainsi que des micro-vibrations, c'est-à-dire des sons, dans une plage de fréquences comprise entre 0,1 Hz et 20 kHz, et pour générer, dans l'entrefer d'air entre le transducteur et la surface du tissu traité, une décharge à barrière diélectrique et un plasma atmosphérique froid faiblement ionisé, l'unité centrale (100) couplée au transducteur (106), lequel comprend en outre un éclateur inductif, générant un champ électromagnétique dans une large plage de fréquences comprise entre 1 Hz et 20 GHz.

11. Dispositif de traitement de maladies et d'états pathologiques (10) selon les revendications 3 à 10, **caractérisé en ce que**, lorsque le dispositif (10) est utilisé pour le traitement d'un tissu, un couplage transducteur (106) - tissu traité (110) - électrode résonante (107) peut être établi, de sorte qu'une voie énergétique résonante thérapeutique (108) peut être établie à travers le tissu cible traité, le bloc générateur et régulateur de profils de signaux (105) mesurant, au moyen dudit couplage, en temps réel, la quantité d'énergie, la tension, le courant et l'impédance pendant le traitement afin de surveiller la quantité d'énergie transmise et la réponse de l'organisme traité à la stimulation, lesquelles dépendent du mode d'administration, du type et de l'état du tissu cible traité et/ou des conditions ambiantes pendant l'application, afin de permettre la commande, en temps réel, de la quantité d'énergie transmise par ajustement des profils d'impulsions de sortie générés par le bloc générateur et régulateur de profils de signaux (105).

12. Dispositif de traitement de maladies et d'états pathologiques (10) selon l'une quelconque des revendications précédentes, **caractérisé en ce que** le transducteur (106) comprend un connecteur (405), un boîtier isolant (401), un élément capacitif et/ou inductif (402), une barrière diélectrique (404) et une source lumineuse (408) configurée pour générer une décharge luminescente dans un volume partiellement évacué délimité par un matériau diélectrique, contenant un ou plusieurs gaz qui, avec l'aide de l'élément capacitif (402), sont couplés au bloc générateur et régulateur de profils de signaux (105), excités à travers la barrière diélectrique (404) par l'intermédiaire du connecteur (405).

13. Dispositif de traitement de maladies et d'états pathologiques (10) selon l'une quelconque des revendications précédentes, **caractérisé en ce que** le transducteur (106) comprend un connecteur (405), un boîtier isolant (401), un élément capacitif et/ou inductif (402), une barrière diélectrique (404), un circuit électronique (407) comprenant une source lumineuse (408) de type LED ou OLED, ou une autre source lumineuse alternative dans la partie ultraviolette, visible ou infrarouge du spectre, excitée par des profils d'impulsions séparés générés par l'unité de gestion (102).

14. Dispositif de traitement de maladies et d'états pathologiques (10) selon les revendications 12 ou 13, **caractérisé en ce que** la barrière diélectrique (404) dans le transducteur (106) est constituée de verre, de céramique ou de polymères.

15. Dispositif de traitement de maladies et d'états pathologiques (10) selon les revendications 12, 13 ou 14, **caractérisé en ce que** l'élément capacitif et/ou inductif (402) est constitué d'un matériau conducteur sous la forme soit d'une plaque capacitive (601), d'un disque (602) ou d'un réseau (603), soit d'un disque inductif (604), soit d'une bobine transmettant la lumière (605), soit d'une bobine segmentée avec un éclateur (606), ou d'une combinaison de ceux-ci, et se trouve du côté proximal de la barrière diélectrique (404).

16. Dispositif de traitement de maladies et d'états pathologiques (10) selon les revendications 12, 13 ou 14, **caractérisé en ce que** l'élément capacitif et/ou inductif (402) est constitué d'un matériau conducteur tel qu'une bobine segmentée avec un éclateur (412), ladite bobine segmentée avec un éclateur comprenant des points de contact pour le couplage à la source (701) et le couplage au récepteur (702), ladite bobine segmentée avec un éclateur (606) pouvant être enroulée de manière plane ou conique, et l'agencement ainsi que la longueur des segments individuels de la bobine plane ou de la bobine enroulée de manière conique présentant une largeur/épaisseur de fil arbitraire d1, intersectée radialement depuis son centre afin de former des espaces (d2) entre les segments individuels de la bobine, et afin d'empêcher la formation d'arcs électriques indésirables entre les spires individuelles de la bobine, l'espace entre les spires individuelles d3 n'étant pas inférieur à deux fois la dimension des espaces d2, et le procédé d'intersection de la bobine étant défini par un cercle divisé en plusieurs segments de telle manière que chaque segment suivant, à partir de l'angle le plus grand, est égal au quotient de l'angle précédent plus grand et de la valeur $\frac{1 + \sqrt{5}}{2}$.

17. Dispositif de traitement de maladies et d'états pathologiques (10) selon les revendications 15 ou 16, **caractérisé en ce que** les éléments inductifs (604), (605), (606) comprennent un point de contact pour le couplage à la source (607) et le couplage au récepteur (608), les éléments inductifs (604), (605), (606) étant directement couplés au bloc générateur et régulateur de profils de signaux (105), le fonctionnement du dispositif (10) étant possible sans contact direct avec l'organisme traité, le transducteur et le dispositif (10) étant positionnés à une certaine distance de l'organisme afin de générer un champ thérapeutique.

18. Dispositif de traitement de maladies et d'états pathologiques (10) selon les revendications 14 à 17, **caractérisé en ce que** la surface active du transducteur (106) sous la forme d'une barrière diélectrique (404), qui est dirigée vers l'organisme traité ou vient en contact avec celui-ci, est plane (801), convexe (802), concave (803) ou pointue (804), ou une combinaison de celles-ci, afin de répondre aux exigences énergétiques et ergonomiques, l'ensemble dépendant du type et des besoins de la procédure thérapeutique, et est constituée de verre, de céramique ou de polymères.

19. Dispositif de traitement de maladies et d'états pathologiques (10) selon les revendications 12 à 18, **caractérisé en ce que** le transducteur (106) comprend des extensions adaptatives (501) destinées au positionnement précis du transducteur (106) par rapport à la surface traitée de l'organisme vivant, et comprend en outre une extension télescopique (507) ayant une paroi interne dont le coulissement permet de définir avec précision la distance entre la surface active du transducteur (barrière diélectrique) et la surface traitée de l'organisme vivant, permettant, pendant le fonctionnement du dispositif (10), de commander les propriétés de la décharge à barrière diélectrique et de retenir les constituants générés par l'apparition d'une décharge à barrière diélectrique dans un volume fermé et précisément défini dont les parois sont constituées par la surface active du transducteur (106), la surface traitée de l'organisme vivant et la paroi interne de l'extension télescopique (507).

20. Dispositif de traitement de maladies et d'états pathologiques (10) selon la revendication 19, **caractérisé en ce que** le transducteur (106) comprend des extensions adaptatives (501) comprenant un filtre photo-diélectrique (508) recouvrant la surface active du transducteur (106) et transmettant un spectre spécifique de lumière vers le tissu cible traité.

21. Dispositif de traitement de maladies et d'états pathologiques (10) selon les revendications 19 ou 20, **caractérisé en ce que** le transducteur (106) comprend des extensions adaptatives (501) contenant un ou plusieurs éléments passifs (509) destinés à maintenir une distance constante entre la décharge à barrière diélectrique et la surface du tissu cible traité.

22. Dispositif de traitement de maladies et d'états pathologiques (10) selon l'une quelconque des revendications précédentes, **caractérisé en ce que** l'électrode résonante (107) est configurée pour venir en contact avec la surface de l'organisme, celle-ci étant constituée d'un matériau conducteur présentant des dimensions et des formes quelconques, et étant couplée à l'unité centrale (100), l'électrode résonante (107) comprenant un circuit électronique configuré pour effectuer des mesures de tension et pour fournir une signalisation visuelle ou sonore afin d'établir un circuit énergétique résonant thérapeutique (108).

23. Dispositif de traitement de maladies et d'états pathologiques (10) selon l'une quelconque des revendications précédentes, **caractérisé en ce que** ledit dispositif est configuré pour générer des profils d'impulsions comprenant des séquences d'impulsions pouvant être adaptées et modulées en fonction de leur forme, laquelle peut être sinusoïdale, rectangulaire, triangulaire, en dents de scie, trapézoïdale, à croissance ou décroissance linéaire ou exponentielle, à harmoniques paires ou impaires, sinusoïdale amortie ou amplifiée exponentiellement, avec une fréquence des séquences d'impulsions comprise entre 0,1 Hz et 7 500 Hz, une fréquence des impulsions modulées comprise entre 20 et 350 Hz, la fréquence de modulation de l'amplitude du signal de modulation par un signal oscillant étant comprise entre 0,1 et 5 040 Hz, l'amplitude des impulsions étant comprise entre environ 500 V et 30 kV, et le champ électromagnétique ainsi que des courants haute fréquence compris entre 40 MHz et 1 GHz et des ondes sonores comprises entre 0,1 et 20 000 Hz étant générés par la génération d'une décharge à barrière diélectrique dans l'entrefer d'air entre la surface du transducteur et la surface du tissu cible traité.

24. Dispositif de traitement de maladies et d'états pathologiques (10) selon l'une quelconque des revendications précédentes, **caractérisé en ce que** ledit dispositif est configuré pour générer des profils d'impulsions comprenant des séquences d'impulsions pouvant être adaptées et modulées en tenant compte de la fréquence des séquences d'impulsions comprise entre 0,1 et 7 500 Hz, de la fréquence des impulsions modulées comprise entre 20 et 350 kHz et de la fréquence du signal de modulation comprise entre 0,1 et 5 040 Hz.

25. Dispositif de traitement de maladies et d'états pathologiques (10) selon l'une quelconque des revendications précédentes, **caractérisé en ce que** la fréquence des séquences d'impulsions est de 0,72 Hz ou l'un quelconque de ses harmoniques, comprenant notamment 1,44 ; 2,16 ; 2,88 ; 3,6 ; 4,32 ; 7,2 ; 14,4 ; 21,6 ; 28,8 ; 36 ; 43,2 ; 50,4 ; 57,6 ; 64,8 ; 72 ; 144 ; 216 ; 288 ; 360 ; 432 ; 504 ; 576 ; 648 ; 720 ; 792 ; 864 ; 936 ; 1 008 ; 1 080 ; 1 152 ; 1 224 ; 1 296 ; 1 368 ; 1 440 ; 2 160 ; 2 880 ; 3 600 ; 4 320 ; 5 040 ; 5 760 ; 6 480 ; 7 200 ; 7 344 Hz, ou un balayage sur la plage de fréquences spécifiée.

26. Dispositif de traitement de maladies et d'états pathologiques (10) selon l'une quelconque des revendications précédentes, **caractérisé en ce que** la fréquence des impulsions modulées est de 21,6 ; 28,8 ; 36 ; 43,2 ; 50,4 ; 57,6 ; 64,8 ; 72 ; 79,2 ; 86,4 ; 93,6 ; 100,8 ; 108 ; 115,2 ; 122,4 ; 129,6 ; 136,8 ; 144 ; 151,2 ; 158,4 ; 165,6 ; 172,8 ; 180 ; 187,2 ; 194,4 ; 201,6 ; 208,8 ; 216 ; 223,2 ; 230,4 ; 237,6 ; 244,8 ; 252 ; 259,2 ; 266,4 ; 273,6 ; 280,8 ; 288 ; 295,2 ; 302,4 ; 309,6 ; 316,8 ; 324 ; 331,2 ; 338,4 ; 345,6 kHz, ou un balayage sur la plage de fréquences spécifiée.

27. Dispositif de traitement de maladies et d'états pathologiques (10) selon l'une quelconque des revendications précédentes, **caractérisé en ce que** la fréquence du signal de modulation est de 0,72 Hz ou l'un quelconque de ses harmoniques, comprenant notamment 1,44 ; 2,16 ; 2,88 ; 3,6 ; 4,32 ; 7,2 ; 14,4 ; 21,6 ; 28,8 ; 36 ; 43,2 ; 50,4 ; 57,6 ; 64,8 ; 72 ; 144 ; 216 ; 288 ; 360 ; 432 ; 504 ; 576 ; 648 ; 720 ; 792 ; 864 ; 936 ; 1 008 ; 1 080 ; 1 152 ; 1 224 ; 1 296 ; 1 368 ; 1 440 ; 2 160 ; 2 880 ; 3 600 ; 4 320 ; 5 040 Hz, ou un balayage sur la plage de fréquences mentionnée.

28. Dispositif de traitement de maladies et d'états pathologiques (10) selon les revendications 25, 26 ou 27, **caractérisé en ce que** la tension au secondaire du transformateur haute tension du bloc générateur et régulateur de profils de signaux (105) est générée sous la forme d'une séquence d'impulsions dont la forme d'onde est sinusoïdale, rectangulaire, triangulaire ou en dents de scie, trapézoïdale, à croissance ou décroissance linéaire ou exponentielle, à harmonique paire ou impaire, sinusoïdale amortie ou amplifiée exponentiellement, lesdites séquences d'impulsions étant modulées par des formes d'onde sinusoïdales, rectangulaires, trapézoïdales, à croissance ou décroissance linéaire ou exponentielle, à harmoniques paires ou impaires, ou sinusoïdales amorties, du signal de commande de tension (signal de modulation) présentant une fréquence comprise entre 0,72 et 5 040 Hz, avec une profondeur d'amplitude de modulation comprise entre 1 et 100 %.
